(19) Europäisches Patentamt / European Patent Office / Office européen des brevets

(11) **EP 4 614 509 A1**

(12) **EUROPEAN PATENT APPLICATION**

(43) Date of publication:
**10.09.2025 Bulletin 2025/37**

(21) Application number: **24382251.7**

(22) Date of filing: **08.03.2024**

(51) International Patent Classification (IPC):
*G16H 30/00* (2018.01)

(52) Cooperative Patent Classification (CPC):
**G16H 30/00;** G16H 30/40; G16H 50/50

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ELEM Biotech S.L.**
**08003 Barcelona (ES)**

(72) Inventor: **POZO SOLER, Jose Maria**
**08003 Barcelona (ES)**

(74) Representative: **Lawrence, Richard Anthony**
**Keltie LLP**
**No.1 London Bridge**
**London SE1 9BA (GB)**

(54) **MUSCULAR FIBRE ESTIMATION**

(57)    A method of estimating muscle fibre architecture for a muscular structure is described. The method comprises receiving a plurality of images that represent the motion of the muscular structure during a motion sequence, receiving an initial model of muscle fibre architecture, receiving a model of mechanical coupling between muscular structure motion and muscle fibres, and generating a joint function using muscular structure motion as represented in the plurality of images, the initial model of muscle fibre architecture, and the model of mechanical coupling. The method further comprises applying an optimisation procedure to optimise the joint function and, from the optimisation procedure, determining a model of muscle fibre architecture consistent with the muscular structure motion indicated in the plurality of images.

Figure 3

EP 4 614 509 A1

**Description**

**Field of the invention**

The present invention relates to estimating and modelling fibre architecture for a muscular structure. Embodiments described in detail relate to myocardial fibre architecture.

**Background of the invention**

Modelling myocardial fibre architecture is of interest in the field of medical research. A realistic heart model provides a platform for simulating the effects of drugs or implanted devices on the heart, and thus help in developing treatments for various heart defects and diseases. However, the myocardial fibre architecture is still not completely understood, and current methods of estimating and modelling myocardial architecture have limited accuracy. Similar problems apply to modelling of other complex muscle structures in the body.

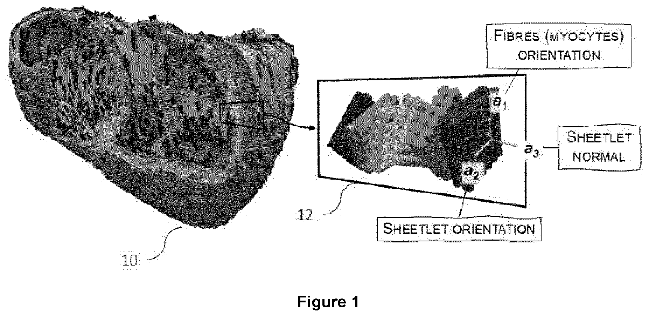

Two aspects are considered when modelling myocardial fibre architecture: the structure of the myocardial fibres themselves and their movement during a heart cycle. The myocardium, the middle layer of the heart muscle, is made up of individual fibres (myocytes) which arrange themselves into sheetlets. This structure is shown in **Figure 1,** which contains a schematic illustration of myocardial fibre and sheetlet architecture in the heart 10, alongside a zoom-in of sheetlets 12 showing the individual myocytes making up the sheetlet. As shown in **Figure 1,** the myocytes locally align with each other along a first orientation within the sheetlet, and the sheetlets then orientate in a second, perpendicular direction. The orientation is important as cardiomyocyte orientation determines the preferential electrical wave propagation and tissue contraction in the heart, and so proper orientation of the myocytes is essential for valid and accurate heart models.

Current methods for determining the orientation of myocardial fibres and creating heart models include simplified geometrical descriptions such as rule-based models (RBMs), and imaging methods such as Diffusion Tensor Imaging (DTI).

Considering firstly RBMs, RBMs assign myocyte orientations using mathematical descriptions based on rules derived from histological and experimental observations. Using the mathematical descriptions, the myocyte orientations are incorporated into a cardiac computational model. However, the resultant model is extremely simplified since the model is based on general biomedical observations. Additionally, this method cannot provide a personalised model of myocardial fibre architecture for a patient, or properly represent any variation in a population, as again, the mathematical rules describing the myocardial fibres are based on general observations. Thus, RBMs essentially provide a general model of the myocardial fibre architecture.

An alternative method for determining myocardial fibre architecture is DTI, which is a widely used imaging modality for assessing tissue microstructure, primarily in the neuroimaging field. DTI captures the anisotropy of water diffusion in a tissue, and so enables determination of individual cell orientation and sheetlet orientation, and thus tissue structure. In more detail, a medical image that is sensitised to diffusion in a particular direction is generated through application of magnetic field gradients to a subject. This process is repeated multiple times, applying the diffusion-encoding magnetic field gradients in different directions, to generate a three dimensional diffusion tensor (DT) for each voxel of the image. The more directions used, the more accurate the resultant tensor. The three eigenvectors ($E_1$, $E_2$, $E_3$) of each diffusion tensor (DT) are then estimated, and the eigenvectors correspond to cell orientation. $E_1$ is the principal eigenvector, that defines the direction of fastest diffusion and thus ideally corresponds to the direction of the fibres in the tissue. When this method is applied to imaging the heart, (cardio (c)DTI), these vectors, $E_1$, $E_2$, $E_3$, correspond to the myocyte orientation, $a_1$, the sheetlet orientation, $a_2$, and the sheetlet-normal direction, $a_3$, respectively, as illustrated **in Figure 1.**

Within cDTI, both *ex-vivo* and *in-vivo* methods exist. While *ex-vivo* cDTI is used for estimating the myocardial architecture, and is more accurate than *in-vivo* cDTI, *ex-vivo* cDTI has the obvious disadvantage of not being applicable to patients or volunteers, reducing it to some post-mortem donors, and thus reducing the available acquisitions to a very small number of cases. If an estimate of the myocardial fibre architecture in a population is required, the small number of cases available would lead to an estimate and model that is not representative of the population. A heart model generated using this method would therefore not be an appropriate model on which to base medical research. In addition, keeping an extracted heart in its natural *in-vivo* configuration corresponding to any typical cardiac phase is extremely challenging. It is therefore impossible to guarantee that the imaged myocardial tissue state corresponds to any *in-vivo* cardiac phase, or to the blood-substitute filling-fluid volume and pressure, which introduces inaccuracies in the interpretation of the estimated fibre architecture.

*In-vivo* cDTI has also emerged in recent years as an alternative method for estimating and subsequently modelling myocardial fibre architecture for an individual patient. For example, Ferreira et al. (Ferreira et al. (2014), In vivo cardiovascular magnetic resonance diffuse tensor imaging shows evidence of abnormal myocardial laminar orientations and mobility in hypertrophic cardiomyopathy, Journal of Cardiovascular Magnetic Resonance, 16(1), 87) describe performing cDTI at two cardiac phases in 22 patients, to measure cross-myocyte diffusion, and identified that patients with hypertrophic cardiomyopathy, demonstrated impaired reorientation of sheetlets in the diastolic phase of the heart cycle. However *in-vivo* cDTI is still an experimental modality, and so is not yet acquired regularly in clinics. Inherent cardiac motion and the resultant deformation of the fibres means that more complex image sequences are required, which leads to a long acquisition time, a high noise level, and a high degree of inaccuracy when acquiring *in-vivo* cardio diffuse tensor images. Indeed, a disadvantage of DTI in general is the low signal to noise ratio, which can affect the accuracy of this technique.

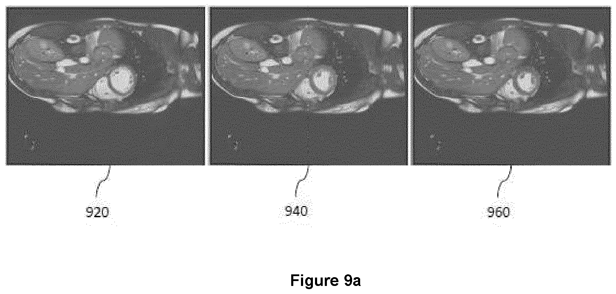

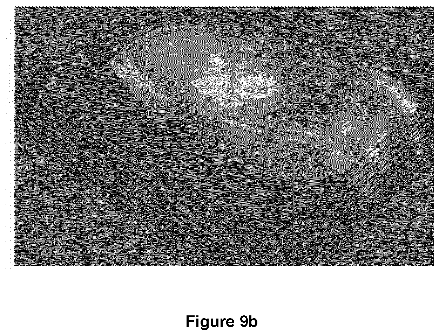

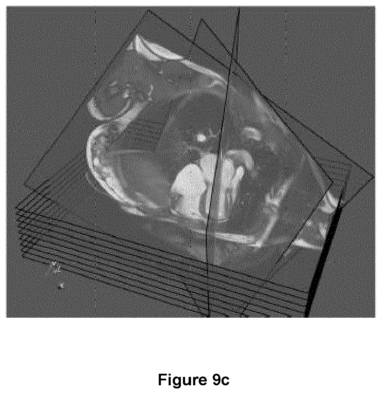

In addition to the above-described disadvantages, neither of the above methods (RBMs and cDTI) take into consideration the movement of the myocardium during the heart cycle as a source of information. Myocardial movement is extremely important as movement leads to deformation of the myocardial fibres, which affects and is affected by fibre structure. Therefore, the response and influence of the fibres on movement is desirable for a realistic, accurate model of the heart. An imaging modality that shows movement is cine-Magnetic Resonance Imaging (MRI). Cine-MRI images are regularly acquired in clinics, and are dynamic images that are able to represent movement of the heart during a complete cardiac cycle. To acquire a cine-MRI image a series of MRI images are acquired in one slice or a stack of parallel slices, over several cardiac cycles, and then the multiple images are rearranged into a series showing a single normalised cardiac cycle. Several cine-MRI image series are typically acquired in different anatomical plane orientations. In general, in the short-axis several MRI slices are acquired at different heights of the heart, which enables them to be visualised as a temporal sequence of 3D images:. **Figure 9a** is an MRI slice shown at a central heart height for three different time points 920, 940, 960, and **Figure 9b** is the same cine-MRI image shown at a single time point for a stack of images at different heart heights. In the long-axis, one slice is typically acquired for each of three cardiac anatomical planes, as illustrated in **Figure 9c,** which also shows the intersection of the long-axis with one slice of the short-axis cine-MRI image.

Therefore, while there are some methods available for estimating heart fibre architecture, and generating models of the myocardium, current methods use limited in their use and accuracy.

It is therefore an aim of the present invention to provide methods for estimating myocardial fibre architecture using readily available, generic cardiac images of the human heart, where the method overcomes some of the limitations discussed above.

## Summary of the invention

In a first aspect, the invention provides a method of estimating muscle fibre architecture for a muscular structure, the method comprising receiving a plurality of images that represent the motion of the muscular structure during a motion sequence, receiving an initial model of muscle fibre architecture, receiving a model of mechanical coupling between muscular structure motion and muscle fibres, and generating a joint function using muscular structure motion as represented in the plurality of images, the initial model of muscle fibre architecture, and the model of mechanical coupling. The method further comprises applying an optimisation procedure to optimise the joint function and, from the optimisation procedure, determining a model of muscle fibre architecture consistent with the muscular structure motion indicated in the plurality of images.

The optimisation procedure may comprise parameterising the muscular structure motion indicated in the plurality of images to determine a set of motion parameters, parameterising the fibre architecture indicated in the initial model of muscle fibre architecture to determine a set of fibre architecture parameters, and optimising the joint function by alternatively or jointly optimising the set of motion parameters and the set of fibre architecture parameters until convergence is reached.

Optimising the set of motion parameters and the set of fibre architecture parameters may comprise calculating the gradient of the joint function with respect to the set of motion parameters and the set of fibre architecture parameters, and applying a gradient - based optimisation algorithm to the joint function.

Determining the model of muscle fibre architecture may comprise using the converged set of motion parameters and set of fibre architecture parameters.

Muscular structure motion may be parameterised using a continuous time-varying velocity field.

Parametrising the muscle fibre architecture may comprise parameterising the local fibre orientation and parameterising the spatial variation in fibre orientation.

The function describing motion, the initial model of muscle fibre architecture and the model of mechanical coupling may be probabilistic models.

The method may additionally comprise generating a conditional probability density function to represent muscular structure motion as indicated in the plurality of images, for the initial model of muscle fibre architecture, and for the model of mechanical coupling. The joint function may be generated using the probability density functions.

The plurality of images may be received from a cine-MRI.

The initial model of muscle fibre architecture may be population-based fibre information provided from rule-based models.

The model of mechanical coupling may be derived from strain properties of muscle fibres.

The method may additionally comprise receiving muscle fibre information for an individual, generating a function describing the muscle fibre information for the individual, and generating the joint function using muscular structure motion as represented in the plurality of images, the initial model of muscle fibre architecture, the model of mechanical coupling, and the function describing the muscle fibre information for the individual.

The muscle fibre information for the individual may comprise at least one diffuse tensor image, and the function describing the myocardial fibre information for the individual may be a conditional probability density function.

The muscular structure may be a heart, the muscle fibre architecture may be myocardial fibre architecture, and the motion sequence may be a cardiac cycle. In such embodiments, the method of estimating myocardial fibre architecture may be used to model the function of a heart.

The muscular structure may be a uterus.

In a second aspect, the invention provides a method of image enhancement in imaging a muscular structure. The method comprises estimating muscle fibre architecture according to the first aspect of the present invention, determining the muscular structure motion during the motion sequence, applying an inverse motion parameter to at least some of the plurality of images to provide images compensated for the muscular structure motion, and combining the compensated images using image fusion or image super resolution to provide at least one higher resolution image of the muscular structure.

In a third aspect, the invention provides a system for estimating muscle fibre architecture using a plurality of images indicating motion of a muscular structure. The system comprises an input engine for receiving each of the plurality of images indicating muscular structure motion, and a function creator configured to generate a joint function using muscular structure motion as represented in the plurality of images, an initial model of muscular fibre architecture, and a model of mechanical coupling between muscular structure motion and muscular fibres. The system further comprises an optimisation engine configured to optimise the joint function, and a model generator configured to determine, using the optimised joint function, a model of muscular fibre architecture consistent with the muscular structure motion indicated in the plurality of images.

## Brief Description of the Drawings

Specific embodiments of the invention will now be described, by way of example, with reference to the accompanying figures, of which:

Figure 1 is a histological image of myocardial sheetlets, alongside a schematic illustration of sheetlets showing the individual myocytes making up the sheetlet;

**Figure 2** is a schematic block diagram showing the overview of an exemplary system for implementing an improved method of modelling myocardial fibre architecture according to embodiments of the present invention;

**Figure 3** is a flow diagram showing an overview of a method used to generate a model of myocardial fibre architecture in accordance with embodiments of the present invention;

**Figure 4** is a flow diagram showing the input processing of Figure 3 in greater detail;

**Figure 5** is a flow diagram showing the optimisation procedure of **Figure 3** in greater detail;

**Figure 6** is a flow diagram showing a method of gradient calculation, in accordance with embodiments of the present disclosure;

**Figure 7** is a flow diagram showing an example gradient descent optimisation algorithm that may be used for the optimisation procedure of **Figure 3;**

**Figure 8** is a schematic diagram showing an example application for the heart model generated using the method of **Figure 3;**

**Figure 9a** is a cine-MRI image acquired in the short axis shown at three time points for a slice at a central heart height;

**Figure 9b** is the same cine-MRI image shown at a single time point for the stack of slices at different heart heights;

**Figure 9c** is a cine-MRI image at one time point, showing MRI images acquired in the long-axis for each of the three typical anatomical planes, and intersecting with one slice of the short-axis cine-MRI image..

**Detailed Description of the Invention**

Prior to setting forth the invention, a number of definitions are provided that will assist in the understanding of the invention. All references cited herein are incorporated by reference in their entirety. Unless otherwise defined, all technical and scientific terms used herein have the same meaning as commonly understood by one of ordinary skill in the art to which this invention belongs.

The terms 'cardiomyocytes', 'cardiac fibres', 'myocardial fibres', 'cardiac fibres' and similar terms are used interchangeably herein and refer to the individual cells which make up the myocardium (the middle layer of heart muscle) of the heart. Cardiomyocytes align with each other and then aggregate to form sheetlets. The cardiomyocytes are responsible for contraction of the heart.

The term 'fibre orientation' does not refer to individual cardiomyocytes, but to the average orientation of the aggregate of cardiomyocytes in the local neighbourhood, which are approximately aligned in the same 3-dimensional orientation. The term 'fibre orientation' also implicitly includes the sheetlet orientation.

The term 'fibre architecture' comprises the local fibre orientation and sheetlet orientation, and their global pattern in the whole myocardium.

**Overview of Method**

The method of generating a heart model according to an embodiment of the present invention comprises estimating cardiac fibre architecture using 2 principal inputs: a plurality of images that represent the myocardial motion during a heart cycle, and an approximation of fibre architecture, provided by a prior probability distribution representing fibre architecture for a population. The method also uses an expression that approximates the mechanical coupling between cardiac motion and myocardial fibres, in order to take into account the effect of fibre structure on cardiac motion. Embodiments describe in detail here relate to modelling of a heart - however, it should be noted that this approach can be applied to other muscular structures in the body, particularly those where motion takes place without the control of the subject (such as the uterus, for example). Where reference is made below to the heart, to myocardial fibres, and to a cardiac cycle, the skilled person will appreciate that the teaching provided may be applied to another muscular structure with its own fibre types and its own motion sequence.

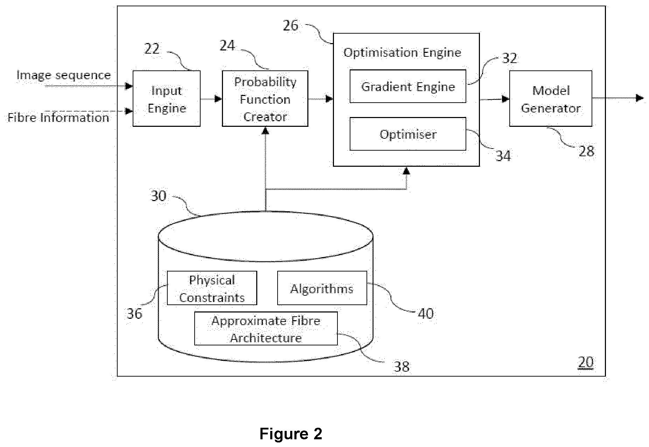

The block diagram in Figure 2 shows the overview of an exemplary system 20 for implementing an improved method of modelling myocardial fibre architecture according to embodiments of the present invention. The system includes an Input Engine 22 operatively coupled to a Probability Function Creator 24. The output of the Probability Function Creator 24 is provided to an Optimisation Engine 26, which comprises a Gradient Engine 32 and an Optimiser 34. The Probability Function Creator 24 and the Optimisation Engine 26 are both in communication with a datastore 30 containing a model for physical constraints 36, a model of an approximate fibre architecture 38, and algorithms 40, each of which will be discussed in greater detail later. The Optimisation Engine 26 is operatively coupled to a Model Generator 28, which outputs a realistic model of the myocardial fibre architecture.

In use, the Input Engine 22 receives subject-specific inputs: an image sequence describing cardiac motion and, optionally, cardiac fibre information for an individual. The image sequence may be, for example, a cine-MRI or a 3D echocardiography, but other image sequences are possible. Several stacks of images or single image slices acquired in different planes (for example, short-axis or long-axis images showing 2, 3, or 4 heart chambers), or acquisitions using different image modalities and temporal resolutions, may be combined. The optional subject-specific fibre information may be obtained, for example, from a cDTI (or from combining several cDTI images acquired at different phases during the heart cycle). It should be noted that subject-specific fibre information, that is a cDTI image for a subject, is an optional input, and in some embodiments this input is not included.

The Input Engine 22 provides the received inputs to the Probability Function Creator 24, which also retrieves the model of physical constraints 36 and the model of approximate fibre architecture 38 from the datastore 30. The Probability Function Creator 24 processes each of the received inputs to provide a log-probability function to the Optimisation Engine 26. The Optimisation Engine 26 optimises the received log-probability function to generate optimised parameters for the fibre architecture. In greater detail, the Gradient Engine 32 calculates the gradient of the log-probability function, and provides the gradient to the Optimiser 34. The Optimiser 34 then retrieves a gradient-based (e.g. gradient-descent) algorithm 40 from the datastore, and applies this algorithm 40 using the calculated gradients, to determine the optimal parameters for fibre architecture, where the optimal parameters are compatible with both fibre architecture and motion (as described in the input image sequence). The optimised parameters are output to the Model Generator 28, which uses the parameters to create a model of myocardial fibre architecture.

Alongside the one or two subject-specific inputs (the image sequence describing motion and fibre information), the Probability Function Creator 24 incorporates two further subject-independent expressions when generating the log-probability function: an approximate model of fibre architecture 38 and a model of physical constraints 36. Considering firstly the approximate model of cardiac architecture 38, the approximate model 38 is constant across all subjects and may be obtained using theoretical (such as RBM) or statistical information on the population. Turning to the second subject-independent expression, the physical constraints 36 are an expression representing the mechanical coupling between cardiac motion and myocardial fibres. The physical constraints 36 are described analytically using equations deduced from the physical properties of the tissues constituting the myocardium, where the equations relate the cardiac deformation and the fibre and sheetlet orientation. Including equations that couple fibres with motion allows for a more accurate estimation of fibre architecture, as it is known that the structure of myocardial fibres affects cardiac motion. The expression for the physical constraints 36 is the same for all subjects.

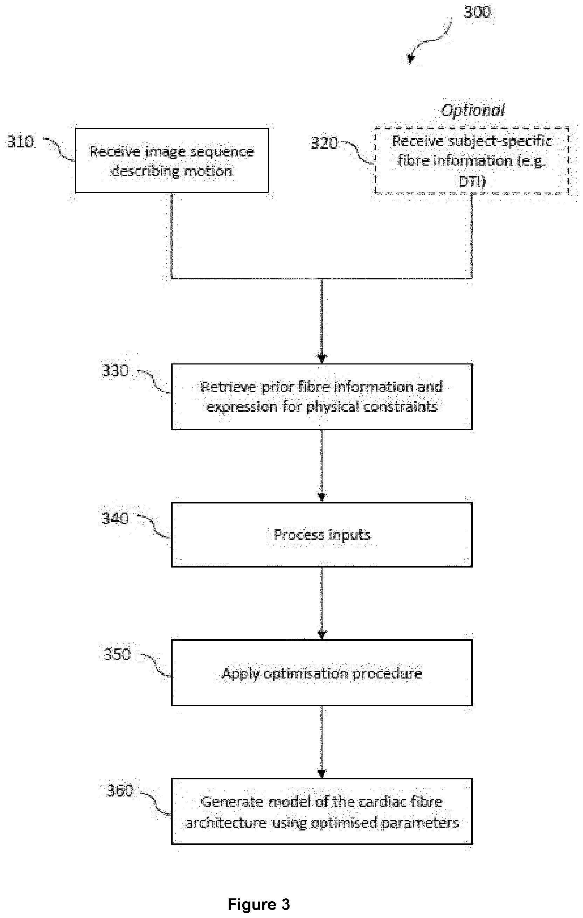

The method 300 carried out by the system 20 and used to generate a model of myocardial fibre architecture according to embodiments of the present invention is shown in the flowchart in **Figure 3.** Firstly, one or several image sequences describing cardiac motion (which is subject-specific) is received at Step 310, and, optionally, subject specific fibre information is received at Step 320. The approximate model 38 of fibre architecture and the expression for the physical constraints 36 is then retrieved at Step 330, and all inputs are processed at Step 340. As described with reference to Figure 2, the input processing results in a log-probability function, and an optimisation function is then applied, at Step 350, to the log-probability function. The optimisation function outputs optimised parameters for the fibre architecture, that are compatible with the cardiac motion observed in the input cine-MRI image sequence. The optimised parameters are used to generate, at Step 360, a model of the cardiac fibre architecture.

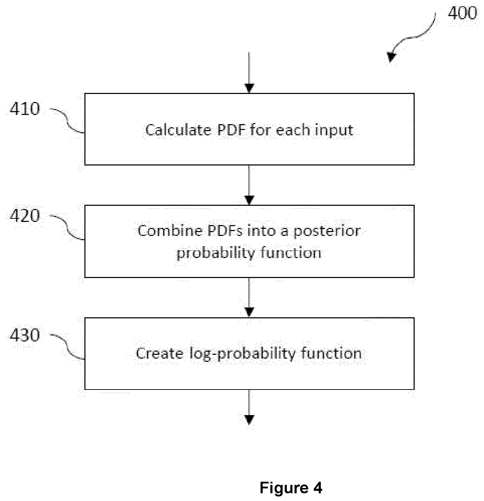

The method of input processing 400, as carried out by the Probability Function Creator 24, is described in greater detail by the flowchart in Figure 4. After receiving the image sequence describing motion and (if available) the subject-specific fibre information, and retrieving the model of approximate fibre architecture 38 and expression for the physical constraints 36, a conditional probability density function (PDF) for each input is calculated at Step 410. The three or four generated PDFs are then combined, at Step 420, into a singular combined posterior probability function (PPF) for the joint distribution of motion and fibre architecture. A log-probability function, which is defined as the negative logarithm of the posterior probability function, is then created at Step 430, and output to the Optimisation Engine 26.

## Calculating Probability Density Functions

As detailed above, the method of generating a model of the heart fibre architecture according to embodiments of the present invention uses two subject-independent expressions (a distribution of fibre architecture, which represents fibre information for a population (from which an approximate model of fibre architecture 38 can be determined), and an expression coupling fibre architecture with cardiac motion) and two subject-specific inputs (an image sequence describing motion and subject-specific fibre information). However, as described above, in some embodiments not all of the inputs are required, and the method may be applied when the subject-specific fibre information is not provided. Equally, in some embodiments, multiple representations of the same input may be provided, for example any number of independent image sequences representing motion or multiple sources of subject-specific fibre information.

As illustrated in Figure 4, a conditional probability density function is calculated for each input, and it should be appreciated any suitable PDF may be used. The derivation of example PDFs that may be used in some embodiments are described below.

In the following PDF calculations several equations show the proportionality of a probability to an expression. The proportionality factor depends only on given inputs, and is independent on the motion and fibres which are meant to be estimated. This makes the proportionality factors irrelevant for the estimation of the motion and fibre parameters, and thus the proportionality factors can be discarded from the resultant PDFs.

## *Image Sequence Describing Motion (subject-specific)*

A required input for the method of the present invention is a sequence of images representing the motion of the heart during a cardiac cycle. A sequence of images $I(x, t)$ is given for a sequence of times $t_0, t_1, ......, t_{n-1}$ during the cardiac cycle. Typically these times are equally spaced ($t_a = aT/n$, where $T$ is the motion period and the times can be considered cyclic ($t_n = t_0$). The sequence of images $I(x, t)$ reflect the motion during a cardiac cycle. Therefore, the conditional probability density function is modelled as having a particular image sequence given a motion: $p(I|\text{Motion})$. Any number and combination of image sequences may be input. In some embodiments, sequences of cine-MRI are considered, however, in other embodiments, other modalities that show motion, such as 3D echocardiography, may be used. An example outlining the derivation of an appropriate PDF $p(I|\text{Motion})$ for an image sequence $I(x, t)$ obtained from a cine-MRI according to an embodiment of the present invention is described in detail below.

Firstly an ideal image sequence $\hat{I}(x, t)$ (an image sequence without noise) is conceptualised as the deformation with motion of an ideal undeformed image $\hat{I}_0(x)$:

$$\hat{I}(x,t) = \hat{I}_0(\chi(x,t,0))$$

**Equation 1**

where $\chi$ is a function representing cardiac motion, so that any pair of images corresponding to times $t_a$ and $t_b$ can be related by the motion between times $t_a$ and $t_b$:

$$\hat{I}(x, t_a) = \hat{I}(\chi(x, t_a, t_b), t_b)$$

**Equation 2**

The real image sequence $I(x, t)$ is modelled as a noisy version of the ideal sequence $\hat{I}(x, t)$, and so the real image sequence $I(x, t)$ satisfies Equation 2 only approximately. A reasonable approximation for cine-MRI images is to model the noise in the real image sequence $I(x, t)$ as additive white Gaussian noise, (other imaging modalities may require a more complex modelling of noise) and thus, the probability density function for the image sequence, given the ideal image sequence and the motion is

$$p(I \mid \hat{I}_0, \text{Motion}) \propto \exp\left[-\frac{1}{2\sigma^2_{im}} \sum_{x,t} (I(x,t) - \hat{I}_0(\chi(x,t,0)))^2\right]$$

**Equation 3**

where $\sigma^2_{im}$ represents image noise, and $\sigma^2_{im} = 0$ describes an image with no noise.

Equation 3 is dependent on the ideal image $\hat{I}_0$, and since the ideal image $\hat{I}_0$ is unknown, this dependency is removed from the conditional PDF. Assuming a naive homogenous prior distribution for $\hat{I}_0$, the joint distribution $p(I,\hat{I}_0|\text{Motion})$, is proportional to the conditional distribution in Equation 3: $p(I,\hat{I}_0|\text{Motion}) \propto p(I \mid \hat{I}_0, \text{Motion})$. To remove $\hat{I}_0$, $\hat{I}_0$ is marginalised by integrating for $\hat{I}_0$. To integrate for $\hat{I}_0$, firstly a change in variables is required: the summation over the voxel positions $x$ is re-expressed as a summation over the undeformed positions $x_0$, which is achieved by approximating the summation by a continuous integral and assuming that the motion is incompressible. It should be noted that without the assumption of incompressible motion, contributions from the Jacobian of the transformation $\chi(x_0, 0, t)$ (from the reference at time 0 to the position at time t of the image, given by the motion between both times) should be incorporated in the probability densities. Expressing the summation over the undeformed voxel positions $x_0$, $p(I,\hat{I}_0|\text{Motion})$ becomes:

$$p(I, \hat{I}_0 \mid \text{Motion}) \propto \exp\left[-\frac{1}{2\sigma^2_{im}} \sum_{x_0,t} (I(\chi_t(x_0), t) - \hat{I}_0(x_o))^2\right]$$

**Equation 4**

where $\chi_t(x_0) = \chi(x_0, 0, t)$.

The integral over $\hat{I}_0$ follows a standard procedure for the marginalisation of multivariate Gaussian distributions, where the result is also Gaussian:

$$p(I|\text{Motion}) \propto \exp\left[-\frac{1}{2\sigma^2_{im}}\left(\sum_{x_0,t} I(\chi_t, (x_0), t)^2 - \frac{1}{n}\sum_{x_0,t_a,t_b} I(\chi_t(x_0), t_a)I\chi_t((x_0), t_b)\right)\right]$$

$$= \exp\left[-\frac{1}{4n\sigma^2_{im}}\sum_{x_0,t_a,t_b}(I(\chi_{t_a}(x_0), t_a) - I(\chi_{t_b}(x_0), t_b))^2\right]$$

**Equation 5**

Assuming again incompressible motion, the summation over points $x_0$ can be approximated by the equivalent summation over points x in any other time,

$$p(I|\text{Motion}) \propto \exp\left[-\frac{1}{4n\sigma^2_{im}}\sum_{t_a t_b}\sum_x (I(x,\ t_a) - I(\chi(x,\ t_a,\ t_b),\ t_b))^2\right]$$

### Equation 6

The exponent is proportional to the typical sum of square error dissimilarity metric between each pair of images in the input image sequence.

It should be noted that in Equation 6 all pairs of images are used to define the PDF. While an embodiment of the present invention calculates the conditional PDF for the image sequence using Equation 6, and each image is compared with every other image in the sequence, in other embodiments a reduced set of comparisons may be used, for example considering only consecutive times: $\{(t_0, t_1), (t_1, t_2), \dots, (t_{n-1}, t_0)\}$, or the first m neighbours: $\{(t_0, t_1, \dots, t_m), (t_1, t_2, \dots, t_{m+1}), \dots, (t_{n-1}, t_0, \dots, t_{m-1})\}$.

***Approximate Model of Fibre Architecture (Population-based fibre information) - Subject-independent***

Population-based prior information 38 on myocardial fibre architecture is also required for the method according to the present invention. Population-based prior fibre information 38 is subject-independent (and so the expression is constant across all subjects), and based on available information of the population. The population-based fibre information 38 can be estimated from any general biological observations on the myocardial fibre architecture, such as the biological or histological data integrated in RBMs or from a statistical atlas built from the fibre architecture of an independent set of representative subjects. The prior information on the fibres provides an initial generic estimate of the myocardial architecture, and thus a probability distribution that reflects the uncertainty in the estimate, and is dependent on the source of the initial estimate $\text{Source}_F$, is modelled $p(\text{Fibre}\ |\ \text{Source}_F)$. The derivation of an example PDF that may be used to model the fibre architecture prior distribution is outlined below, but again, any suitable PDF may be used.

As described previously, the myocardial fibre architecture comprises local myocytes oriented in an approximately parallel direction, which then aggregate into planar sheetlets. Thus the myocardial fibre architecture can be modelled as a field of frames (orthonormal triads) $\{a_1, a_2, a_3\}$, which are defined in the undeformed myocardium (at the reference configuration, $t = 0$). The first vector, $a_1$, represents the orientation direction of the myocardial fibres, the second vector, $a_2$, represents the orientation direction of the sheetlet, perpendicular to the fibres, and the third vector, $a_3$, represents the normal to the sheetlet (as shown in Figure 1).

A fibre frame at a particular point may be parametrised in different ways, including by angle and axis of rotation, by quaternions, or by Euler angles. Representing a frame at a particular point using Euler angles is analogous to the parameterisation used in RBMs, where the fibre frame is given by the three angles, helical $(\alpha_{he})$, transverse $(\alpha_{tr})$, and sheet $(\beta)$. These three angles represent rotations aligned with and applied to a heart-adapted fixed reference frame, $\{e_c, e_l, e_t\}$ (as described in Bayer, J. D., Blake, R. C., Plank, G., & Trayanova, N. A. (2012). A novel rule-based algorithm for assigning myocardial fiber orientation to computational heart models. Annals of biomedical engineering, 40, 2243-2254), denoting the circular, longitudinal, and transmural directions following known conventions.

The spatial variation of the field of frames can also be parametrised in different ways: as a dense field with some regularisation or with a lower dimensional set of parameters based, for example, on splines. In the example discussed below, Euler angles and 3D b-splines on the adapted coordinates of the myocardium (circular, longitudinal, and transmural), (as described in Bayer, J., Prassl, A. J., Pashaei, A., et. al. (2018). Universal ventricular coordinates: A generic framework for describing position within the heart and transferring data. Medical image analysis, 45, 83-93) are used to parameterise the field of frames.

Using Euler angles and B-splines, the field of frames $\{a_1, a_2, a_3\}$ are expressed as a function of a set of spline parameters: $\{a_i(x; \{\omega^A\})\}$. The probability distribution of the fibres is therefore also expressed in terms of the spline parameters:

$$p(\text{Fibre}|\text{Source}_F) = p(\{\omega^A\}|\text{Source}_F)$$

### Equation 7

As shown in Equation 7, the probability distribution depends on the information available from the $\text{Source}_F$, and a typical and versatile model is a multivariate Gaussian distribution on the parameters:

$$p(\text{Fibre} \mid \text{Source}_F) \propto \exp\left(-\frac{1}{2}\left(\omega^A - \overline{\omega^A}\right) P_{AB} \left(\omega^B - \overline{\omega^B}\right)\right)$$

## Equation 8

where the mean parameters' value, $\overline{\omega^A}$ and precision matrix, $P_{AB}$, depend on $\text{Source}_F$.

***Physical Constraints - Subject Independent***

The method according to the present invention also uses an expression for the set of physical constraints representing the coupling between cardiac motion and myocardial fibre architecture. The expression for the physical constraints 36 is subject-independent and based on an approximate modelling of the mechanical relationship between local fibre orientation and tissue deformation given by the motion. This coupling is not deterministic but modelled as a conditional probability density, $p(\text{Motion} \mid \text{Fibres})$. The derivation of an example PDF is outlined in detail below, including first the derivation of a set of physical constraints 36.

The physical constraints 36 are related to fibre-motion coupling, and thus firstly, the transformation of myocardial fibres with motion is considered. This transformation can be formulated in different ways, and one example is described below for illustrative purposes.

The field of frames (orthonormal triads) $\{a_1, a_2, a_3\}$ representing the fibre architecture of the undeformed myocardium (at t=0) do not propagate with motion as vectors. The field of frames propagate in a complex way, however, for simplicity, their vectorial propagation can be considered as an auxiliary field:

$$\tilde{a}_i(x_t, t) = J(x_0, t) a_i(x_0)$$

## Equation 9

where $x_t = \chi_t(x_0) = \chi(x_0, 0, t)$ denotes the motion from the undeformed configuration to the deformed configuration at time t, and $J(\omega^A, t)$ is the Jacobian tensor of the transition. To obtain the motion value at a given point $x_t$ in the deformed tissue at time $t$, the inverse transformation is required: $x_0 = \chi_t^{-1}(x_t) = \chi(x_t, t, 0)$ .

The auxiliary field defined in Equation 9 is not orthonormal, and does not properly represent the deformation of the fibre architecture. However, given the hierarchical definition of the orientation directions of myocardial fibres, an appropriate transformation can be obtained by the Gram-Schmidt orthonormalization of the auxiliary field in the hierarchy order, which is that the first vector $a_1$ represents fibre axis, the second vector $a_2$ represents the sheetlet plane, and the third vector $a_3$ represents the sheetlet normal. Equations 10 and 11 below show the orthonormalization method used:

$$\breve{a}_1 = \tilde{a}_1, \qquad \breve{a}_2 = \tilde{a}_2 - (\tilde{a}_2 \cdot a_1)a_1, \qquad \text{and} \quad \breve{a}_3 = \tilde{a}_3 - (\tilde{a}_3 \cdot a_1)a_1 - (\tilde{a}_3 \cdot a_2)a_2$$

## Equation 10

Where $\breve{a}_i$ represent intermediate vectors which are orthogonal but not unit vectors (normalised). The fibre frames transformed by the motion $a_i$ are obtained by normalisation of the intermediate vectors:

$$a_i, = \frac{\breve{a}_i}{\|\breve{a}_i\|}$$

## Equation 11

The physical constraints 36 used in embodiments of the present invention are derived from the important physical characteristic that there is highly rigid connectivity between fibres in the same sheetlet compared with the easy sliding between sheetlets. This property is part of the known mechanical properties of the myocardium, and is reflected in the very small shear strain expected on the sheetlet plane in comparison with any transversal direction. These physical constraints 36 can be expressed using the Green-Lagrangian strain tensor $E$, and its projections $\varepsilon_{12}$ to the undeformed fibre frame:

$$\varepsilon_{12}(\boldsymbol{x_0}, t) = \boldsymbol{a_1}(\boldsymbol{x_0})^\top \boldsymbol{E}(\boldsymbol{x_0}, t)\boldsymbol{a_2}(\boldsymbol{x_0})$$

**Equation 12**

Thus, the ideal deformation with shear between sheetlets occurs if $\varepsilon_{12}(x_0, t) = 0$. This equation implies a constraint between motion and fibres.

Expressing the Green-Lagrangian strain tensor $\boldsymbol{E}$ in terms of the Jacobian tensor $\boldsymbol{J}$, the model for physical constraints 36 described in Equation 12 becomes

$$\varepsilon_{12}(\boldsymbol{x_0}, t) = \frac{1}{2}\boldsymbol{a_1^T}(\boldsymbol{J^T J} - \mathbb{1})\boldsymbol{a_2} = \frac{1}{2}\boldsymbol{a_1^T J^T J a_2} = \frac{1}{2}\big(\boldsymbol{J}(\boldsymbol{x_0}, t)\boldsymbol{a_1}(\boldsymbol{x_0})\big) \cdot \big(\boldsymbol{J}(\boldsymbol{x_0}, t)\boldsymbol{a_2}(\boldsymbol{x_0})\big)$$

**Equation 13**

where $\boldsymbol{a_1} \cdot \boldsymbol{a_{2q}} = 0$. The physical constraints 36 as described in Equation 13 can be expressed with the auxiliary field defined by the vectorial propagation (Equation 9 above):

$$\varepsilon_{12}(\boldsymbol{x_0}, t) = \frac{1}{2}\tilde{a}_1(x_t, t) \cdot \tilde{a}_2(x_t, t)$$

**Equation 14**

In some embodiments, quasi-incompressibility of the myocardium may also be considered: the volume change that arises from deformation may be encoded by the determinant of the Jacobian tensor, det $\boldsymbol{J(x_0, t)} = 1$, or by the divergence of the Eulerian velocity field of the motion $\nabla \cdot v(x, t) = 0$. However, incorporating this property is optional, and is not discussed in detail.

As the model of the physical constraints 36 described by Equation 14 is an approximation, a probabilistic description of the constraints is used. A Gaussian-like conditional probability is considered for the motion given the fibre architecture:

p(Motion | Fibres)

$$\propto \exp\left[-\frac{1}{2\sigma_{co}^2}\frac{1}{T}\int_0^T \mathrm{d}t\left(\sum_{x_0}\big(\big(\boldsymbol{J}(\boldsymbol{x_0}, t)\boldsymbol{a_1}(\boldsymbol{x_0})\big) \cdot \big(\boldsymbol{J}(\boldsymbol{x_0}, t)\boldsymbol{a_2}(\boldsymbol{x_0})\big)\big)^2\right.\right.$$
$$\left.\left.+ \frac{T^2}{V_{\text{Myoc}}}\int_{\text{Myoc}} d^3\boldsymbol{x}\left(\boldsymbol{\nabla} \cdot \boldsymbol{v}(\boldsymbol{x}, t)\right)^2\right)\right]$$

**Equation 15**

In Equation 15 $\sigma_{co}$ is the constraint standard deviation, and so indicates how precise the physical constraints 36 are expected to be. For an exact constraint, $\sigma_{co} \to 0$, but as the expression for the physical constraints 36 is an approximation, $\sigma_{co} > 0$. The inclusion of the motion period T and the myocardium volume $V_{\text{Myoc}}$ ensures that $\sigma_{co}$ is dimensionless. The $\sigma_{co}$ is expected to be small.

*Fibre Information - Subject-specific (Optional)*

In some embodiments subject-specific fibre information, for example a cDTI, (or any similar image modality that allows extraction of the local fibre orientation) is also included, and used to generate the model of myocardial fibre architecture. This input is optional, however it is advantageous to incorporate this input if available as a subject-specific cDTI provides specific information on local fibre orientation for a subject, and this information improves the precision and accuracy of the estimated fibre architecture. Any number of cDTI images can be combined for this input, including acquisitions in different slices, plane orientations and cardiac phases (such as atrial diastole, ventricular diastole, among many others).

The available fibre images are described by a conditional probability, $\boldsymbol{p}(\textbf{DTI} \mid \text{Fibre, Motion})$, where the images are dependent on the fibre architecture and myocardial motion. It should be appreciated that if the DTI are acquired at a single cardiac phase taken as reference, there is no dependence on the motion. However, for DTI acquired at several cardiac phases, the dependence on the motion cannot be neglected, since, as described above (Equations 9 to 11), the fibre frame at time $t$, $a_i(x_t, t)$, depends on the fibres at the reference configuration, $a_i(x_0)$, and the motion, $\chi_t(x_0)$.

Multiple DTI can be used in some embodiments of the present invention. When a series of DTI at different cardiac phases are present, the estimation of the fibres incorporates the information present in all of the images in a single fibre model.

The derivation of an example PDF that may be used when DTI are acquired at several cardiac phases, and thus there is dependence on motion, is outlined below.

At each voxel/pixel of the DTI, the ideal diffusion tensor $D$ is expected to have as eigenvectors the fibre frame:

$D = \sum_{i=0}^{3} \lambda_i\, a_i \otimes a_i$ , with diffusivity eigenvalues in decreasing order, $\lambda_1 \geq \lambda_2 \geq \lambda_3 \geq 0$. However, the acquired image will be affected by noise, resulting in a probability distribution of the measured diffusion tensor around the ideal one. This tensorial probability distribution is complex but a practical approximation used in the method according to the present invention is a Gaussian distribution with isotropic variance $\sigma_{\mathrm{DTI}}^2$ for all tensor components:

$$p(D|\{a_i\}) \propto \exp\left[-\frac{1}{2\sigma_{\mathrm{DTI}}^2}\left(D^{jk} - \sum_i \lambda_i\, a_i{}^j\, a_i{}^k\right)\left(D_{jk} - \sum_i \lambda_i\, a_{ij}\, a_{ik}\right)\right]$$
$$= \exp\left[-\frac{1}{2\sigma_{\mathrm{DTI}}^2}\left(2D^{jk}D_{jk} - 2\sum_i \lambda_i\, a_i{}^j\, a_i{}^k\, D_{jk}\right)\right]$$
**Equation 16**

where the second expression results from 2 characteristics: the frame $\{a_i\}$ is orthonormal, and $\sum_i \lambda_i^2 = D^{jk}D_{jk}$. In addition, $D^{jk}D_{jk}$ is independent of the fibre frame $\{a_i\}$. Thus, the $D^{jk}D_{jk}$ term does not affect the fibres estimation and can be disregarded as a constant factor, so that the relevant proportional part is

$$p(D|\{a_i\}) \propto \exp\left[\frac{1}{\sigma_{\mathrm{DTI}}^2} \sum_i \lambda_i\, a_i{}^j\, a_i{}^k\, D_{jk}\right]$$
**Equation 17**

Equation 17 is a matrix Bingham distribution (a normal distribution for the space of orthonormal frames). This is an antipodally symmetric probability distribution, in agreement with the property that fibres and sheetlets have no defined sense or sign, so that $\pm a_i$ represent the same direction.

Equation 17 models the local fibre orientation at one point and time $\{a_i(x_t, t)\}$ corresponding to the diffusion tensor at one pixel in one DTI image. Therefore, the complete probability density involves the summation for all DTI pixels:

$$p(\mathrm{DTI}\,|\,\mathrm{Fibres}, \mathrm{Motion}) \propto \exp\left[\frac{1}{\sigma_{\mathrm{DTI}}^2} \sum_t \sum_x \sum_i \lambda_i(x_t, t)\, a_i{}^j(x_t, t)\, D_{jk}(x_t, t)\, a_i{}^k(x_t, t)\right]$$
**Equation 18**

It should be appreciated that the points over which the summation is performed are the DTI pixels where information on the fibres can be extracted. Thus, the points do not coincide, in general, with the voxels over which summation was performed on the image sequence $I(x, t)$ (Equation 6). For instance, if the fibre information is extracted using only a few cDTI slices, the summation should run through the pixels segmented as myocardium on those DTI slices.

The four probability density functions described above are example PDFs that may be used for each input, but as outlined, any other appropriate PDF may be used.

## Joint Posterior Probability Density Function

Returning to Figure 4, as illustrated Step 2 of input processing comprises combining the four (or three if subject-specific fibre images are not included) conditional probability density functions into a singular posterior joint probability of motion and fibres, which is conditional to the input image sequence, DTI, and the source of population-based fibre information:

$$p(\text{Motion}, \text{Fibres}|\text{I}, \text{DTI}, \text{Source}_F) \propto p(\text{Motion}, \text{Fibres}, \text{I}, \text{DTI}| \text{Source}_F)$$

$$= p(\text{I}, \text{DTI}|\text{Motion}, \text{Fibres}) \times p(\text{Motion}, \text{Fibres}| \text{Source}_F)$$

$$= p(\text{I}|\text{Motion}) \times p(\text{DTI}|\text{Fibres}, \text{Motion}) \times p(\text{Motion}|\text{Fibres})$$

$$\times p(\text{Fibres}| \text{Source}_F)$$

### Equation 19

Equation 19 is simply the product of the four probability density functions described previously. In deriving Equation 19, three properties involving conditional independence have been used. First, the image sequences and DTI are conditionally independent once fibres and motion are given:

$$p(I, \text{DTI}|\text{Fibres}, \text{Motion}) = p(\text{I}|\text{Fibres}, \text{Motion}) \times p(\text{DTI} \mid \text{Fibres}, \text{Motion})$$

### Equation 20

Second, the image sequence is conditionally independent of the fibres once the motion is given:

$$p(\text{I}|\text{Fibres}, \text{Motion}) \times p(\text{I} \mid \text{Motion})$$

### Equation 21

Third, the motion is independent of the population-based prior fibre information once the fibres are given:

$$p(\text{Motion}|\text{Fibres}, \text{Source}_F) = p(\text{Motion} \mid \text{Fibres})$$

### Equation 22

In embodiments where no subject-specific DTI are available, and the method only uses three inputs (an image sequence describing motion, an expression for the physical constraints of the fibres 36, and an approximate model of fibre architecture 38) the method is still applicable and the posterior probability function is simplified to:

$$p(\text{Motion}, \text{Fibres}|\text{I}, \text{Source}_F) \propto p(\text{I}|\text{Motion}) \times p(\text{Motion}|\text{Fibres}) \times p(\text{Fibres} \mid \text{Source}_F)$$

### Equation 23

The two Posterior Probability Functions described in Equations 19 and 23 are simple cases of hierarchical Bayesian models. Typically, Bayesian estimation uses Gibbs posterior sampling, however the present invention involves motion tracking (that is, heart movement), which is better modelled as an optimisation problem. Therefore, the algorithm for optimising the fibre architecture parameters is developed as a maximum posterior estimation. It should be appreciated however that in some embodiments, Gibbs posterior sampling may be used to optimise the Posterior Probability Functions.

### Log-probability Function

Determining the maximum of the joint posterior probability function is equivalent to determining the minimum of the negative logarithm of the posterior probability function, so named the log-probability function. Thus, as illustrated in Figure 4, the final step of input processing comprises creating a log-probability function. The derivation of two example log-probability functions, using the posterior probability functions in Equations 19 and 23, are outlined below:

when subject-specific DTI are provided:

-log $p$ (Motion, Fibres | I, DTI, Source$_F$) = -log $p$ (I|Motion) - lop $p$ (DTI|Fibres, Motion) - log $p$(Motion|Fibres) - log $p$(Fibres|Source$_F$) + constant

Equation 24

Or, when no subject-specific fibre information is provided

-log $\boldsymbol{p}$ (Motion, Fibres | I, Source$_F$) = -log $\boldsymbol{p}$ (I|Motion) - lop $\boldsymbol{p}$ (Motion|Fibres) - log $\boldsymbol{p}$(Fibres|Source$_F$) + constant

Equation 25

The constant in Equations 24 and 25 is dependent on the inputs but independent of the fibre and motion parameters that are to be estimated. Thus, the constant is irrelevant for this estimation (the optimal is the same irrespective of the value of this constant) and is therefore ignored when computing the negative logarithm of the posterior probability function.

The resulting expression for the log-probability function is a summation of positive defined metrics, most of which are quadratic. Substituting Equation 24 with the negative logarithm of the PDFs described in Equations 6, 18, 15, and 8, the log-probability function becomes:

$$- \log p(\text{Motion, Fibres | I, DTI, Source}_{\mathbf{F}})$$

$$= \frac{1}{4n\sigma_{\text{im}}^2} \sum_{t_a t_b} \sum_{\boldsymbol{x}} (I(\boldsymbol{x}, t_a) - I(\chi(\boldsymbol{x}, t_a, t_b), t_b))^2$$

$$+ \frac{1}{\sigma_{\text{DTI}}^2} \sum_{t} \sum_{\boldsymbol{x}} \sum_{i} \lambda_i(\boldsymbol{x}_t, t) \, a_i{}^j(\boldsymbol{x}_t, t) \, D_{jk}(\boldsymbol{x}_t, t) \, a_i{}^k(\boldsymbol{x}_t, t)$$

$$+ \frac{1}{2n\sigma_{\text{co}}^2} \sum_{t} \left( \sum_{\boldsymbol{x_0}} \left( (\boldsymbol{J}(\boldsymbol{x_0}, t)\boldsymbol{a}_1(\boldsymbol{x_0})) \cdot (\boldsymbol{J}(\boldsymbol{x_0}, t)\boldsymbol{a}_2(\boldsymbol{x_0})) \right)^2 \right.$$

$$\left. + \frac{T^2}{N_{\text{Myoc}}} \sum_{\boldsymbol{x}} (\boldsymbol{\nabla} \cdot \boldsymbol{v}(\boldsymbol{x}, t))^2 \right) + \frac{1}{2} \left( \omega^A - \overline{\omega^A} \right) P_{AB} \left( \omega^B - \overline{\omega^B} \right)$$

**Equation 26**

The first term in Equation 26 relates to the image sequence representing motion, the second term to subject-specific fibre information, the third term to the set of physical constraints 36, and the final term to the population-based fibre information 38. In embodiments where no subject-specific fibre information is provided, the second term in the equation (-log $\boldsymbol{p}$ (DTI | Fibres, Motion), is not included.

The precision of the maximum-posterior estimates (corresponding to the minimum of the log-probability function obtained using Equation 26) can be quantified by the second derivatives (Hessian matrix) of Equation 26, but this will not be described in detail here.

**Optimisation Procedure**

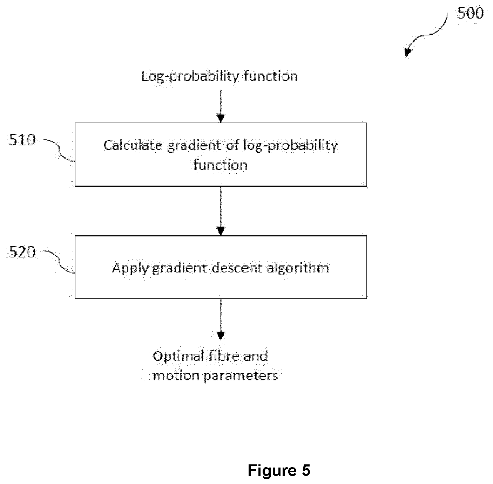

The optimisation method 500 used in embodiments of the present invention, where the minimum of the log-probability function is determined, is illustrated by the flowchart in Figure 5.

Once the log-probability function is generated, the gradient of the log-probability function is calculated, at Step 510, with respect to both the fibre and motion parameters. The resultant gradients are then used to apply, at Step 520, a gradient descent algorithm, which uses an alternation of partial gradient descents for each parameter type (fibre and motion) in order to find the minimum of the log-probability function. Gradient descent is an iterative optimisation algorithm for finding a local minimum of a differentiable function, and so this optimisation method is well-suited to the present application. The output of the gradient descent algorithm is a set of optimised parameters representing motion and fibre architecture, which can be used to construct a heart model. The resultant optimised parameters for the fibre architecture will be compatible with the motion observed in the input image sequence, and thus will provide a heart model with increased accuracy compared to models generated using prior art methods.

**Gradient Calculation**

As described above, to determine the minimum of the log-probability function, the gradient of the log-probability function must be calculated. Since the log-probability function (Equation 26) is the sum of different terms, the gradient is the sum of the gradient of each term, and thus the gradient of each term is calculated separately.

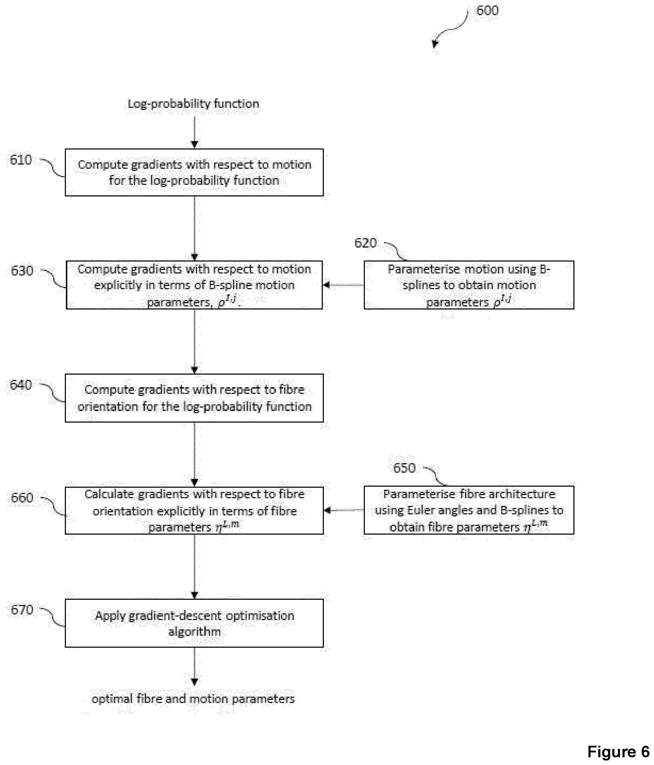

The method of gradient calculation 600 is described in the flowchart in Figure 6. Firstly, an expression for the gradient with respect to the motion parameters for each term in the log-probability function is determined at Step 610. This includes the gradient of image dissimilarity with respect to motion in the input image sequence, and the gradient of the fibre-motion coupling (physical constraints term). If subject-specific DTI is available in different cardiac phases, the gradient of the corresponding term with respect to motion is also included. It should be noted that at this stage, the motion parameters are not concreted, and are generically denoted by $\theta = \{\theta^A\}$. The motion is then parameterised, at Step 620, using B-splines to obtain an iterative formula that parameterises the motion using parameters $\rho^{l,j}$. It should be noted that while this is shown as occurring after expressions for the gradient with respect to motion parameters are obtained, parameterising the motion can occur in parallel with or before determining expressions for the gradient. The motion parameters obtained at Step 620 are then used in conjunction with the gradient expressions determined at Step 610 to calculate, at Step 630, the gradient of each term with respect to motion explicitly using the motion parameters $\rho^{l,j}$. It should also be noted that explicit calculation using motion parameters may not be required for computing the gradient of every term in a function. For example, in Equation 45 (the gradient of motion with respect to motion parameters) only the first term requires explicit motion parameters $\rho^{l,j}$, This is useful when the method is repeated many times (for example, receiving inputs from multiple different subjects), as it means the method requires less computational power.

An expression for the gradient with respect to the myocardial fibres for each term in the log-probability function is then determined at Step 640. At this stage the myocardial fibres are described generally, and in some embodiments this method step may occur before or in parallel with determining the gradients with respect to motion. The fibre architecture is parameterised, at Step 650, using Euler angles with spatial dependency parameterised in turn by B-splines on myocardium adapted coordinates. Thus, the gradients determined at Step 640 are calculated explicitly using the fibre B-spline parameters $\eta^{L,m}$ at Step 660. Once all gradients are calculated, the gradient descent algorithm is applied at Step 670 in order to determine the minimum of the negative log-probability function, and thus determine the optimal parameters for generating a model of the myocardial architecture.

Details on how each of these gradients may be calculated, as well as an example optimisation algorithm, is described in detail below.

### Gradient with respect to motion

As illustrated in Figure 6, an expression for the gradient of each term in the log-probability function with respect to motion parameters is determined. At this stage, the motion is considered to be parametrised by a set of general parameters $\theta = \{\theta^A\}$. The dependence on motion appears in three terms of the log-probability function: $\log p(I \mid \text{Motion})$, $\log p(\text{Motion} \mid \text{Fibres})$, and $\log p(\text{DTI} \mid \text{Fibres, Motion})$, if included (relating to the image sequence describing motion, the expression for physical constraints 36, and the subject-specific fibre information respectively).

The first of these terms is the image dissimilarity, relating to the input sequence of images that represent cardiac motion. Computing the gradient with respect to motion of the image dissimilarity term in the negative log-probability function (Equation 26) gives:

$$-\frac{\partial \log p(I \mid \text{Motion})}{\partial \theta^A}$$

$$= \frac{1}{2n\sigma_{\text{im}}^2} \sum_{t_a t_b} \sum_{x} \big( I(x, t_a)$$

$$- I(\chi(x, t_a, t_b), t_b) \big) \nabla_i I(\chi(x, t_a, t_b), t_b) \frac{\partial \chi^i(x, t_a, t_b)}{\partial \theta^A}$$

**Equation 27**

Two new factors appear compared to Equation 26: the gradient of the image with respect to position, $\nabla_i I(x, t)$ (which is computed numerically using finite differences or an appropriate convolutional filter, as is typical in the field), and the gradient of the motion with respect to the motion parameters, $\frac{\partial \chi^i(x, t_a, t_b)}{\partial \theta^A}$.

The term representing the image dissimilarity, $-\log p(I \mid \text{Motion})$, is the most computationally expensive term when computing the gradient. For this term, all pairs of images are considered:

$$P = \{(a, b) \mid a, b \in [0, n - 1], a \neq b)\}$$

**Equation 28**

However, in some embodiments the method may begin with a relaxed objective function, considering a subset of pairs, which can be progressively increased in a sequence, $P_0 \subset P_1 \subset \ldots \subset P$, defining stages of the optimisation algorithm, analogous to a usual multiresolution cascade. This method improves both the computational efficiency and the stability of the function. For the initial subset of image pairs, only consecutive times in a cycle are considered

$$P_0 = \{(0,1),(1,2),\ldots,(n-2),(n-1,0)\}$$

**Equation 29**

Initially considering consecutive times in some embodiments is advantageous as consecutive images are the most similar, and so the gradients obtained from each of the pairs are more accurate estimates of the direction of optimisation. After several iterations of the optimisation algorithm, all warped images are expected to be close enough to work similarly well for the next iterations. The inclusion of more distant pairs of images is expected to refine the motion extracted from the plurality of images, increasing the stability to image noise, and reducing cumulative errors in the full cardiac cycle motion from small over/under-estimations of the motion between consecutive pairs.

The second term in the log-probability function that is dependent on motion is the expression representing the fibre-motion coupling (the physical constraints 36 of the fibre architecture) $\log p$ (Motion| Fibres). Computing the gradient for this term with respect to motion gives:

$$-\frac{\partial \log p(\text{Motion}|\,\text{Fibres})}{\partial \theta^A}$$

$$= \frac{1}{n\sigma_{\text{co}}^2} \sum_t \left( \sum_{x_0} 2 \left( (J(x_0,t)a_1(x_0)) \cdot (J(x_0,t)a_2(x_0)) \right) \left( J(x_0,t)a_{(1}(x_0) \right) \right.$$

$$\left. \cdot \left( \frac{\partial J(x_0,t)}{\partial \theta^A} a_{2)}(x_0) \right) + \frac{T^2}{N_{\text{Myoc}}} \sum_x (\nabla \cdot v(x,t)) \frac{\partial \nabla \cdot v(x,t)}{\partial \theta^A} \right)$$

**Equation 30**

Two gradients of motion derived quantities appear: the gradient of the Jacobian tensor, $\frac{\partial J(x_0,t)}{\partial \theta^A}$, and the gradient of the divergence of the velocity field, $\frac{\partial \nabla \cdot v(x,t)}{\partial \theta^A}$.

The third term dependent on motion is, if included, the subject-specific DTI fibre information. The gradient of this term with respect to motion only appears when DTI is available for more than one cardiac phase. When only a single cardiac phase is available, this term is not included.

Computing the gradient of the DTI term in Equation 26 with respect to motion gives:

$$-\frac{\partial \log p(\text{DTI}\,|\,\text{Fibres},\text{Motion})}{\partial \theta^A} = \frac{2}{\sigma_{\text{DTI}}^2} \sum_t \sum_x \sum_i \lambda_i(x_t,t)\, a_i{}^j(x_t,t) D_{jk}(x_t,t) \frac{\partial a_i{}^k(x_t,t)}{\partial \theta^A}$$

**Equation 31**

One new gradient factor has appeared: the gradient of the transformed fibre orthonormal frame, $\frac{\partial a_i{}^k(x_t,t)}{\partial \theta^A}$. Given the frame definition above (Equations 10 and 11), by orthonormalizing the vector transformation of the frame in the undeformed reference time, the gradient of the transformed frame with respect to motion can be expressed in terms of the angular velocity tensor $\omega_i$

$$\frac{\partial a_1}{\partial \theta^A} = -\omega_3 a_2 + \omega_2 a_3,$$

**Equation 32**

$$\frac{\partial \boldsymbol{a}_2}{\partial \theta^A} = \omega_3 \boldsymbol{a}_1 - \omega_1 \boldsymbol{a}_3,$$

**Equation 33**

$$\frac{\partial \boldsymbol{a}_3}{\partial \theta^A} = -\omega_2 \boldsymbol{a}_1 + \omega_1 \boldsymbol{a}_2$$

**Equation 34**

using

$$\omega_1 = \frac{1}{\|\tilde{\boldsymbol{a}}_2\|} \left( (\boldsymbol{a}_1 \cdot \tilde{\boldsymbol{a}}_2) \omega_2 - \boldsymbol{a}_3 \cdot \frac{\partial \tilde{\boldsymbol{a}}_2}{\partial \theta^A} \right),$$

**Equation 35**

$$\omega_2 = \frac{1}{\|\tilde{\boldsymbol{a}}_1\|} \left( \boldsymbol{a}_3 \cdot \frac{\partial \tilde{\boldsymbol{a}}_1}{\partial \theta^A} \right),$$

**Equation 36**

$$\omega_3 = -\frac{1}{\|\tilde{\boldsymbol{a}}_1\|} \left( \boldsymbol{a}_2 \cdot \frac{\partial \tilde{\boldsymbol{a}}_1}{\partial \theta^A} \right)$$

**Equation 37**

where, from Equation 9, $\frac{\partial \tilde{\boldsymbol{a}}_i(\boldsymbol{x}_t, t)}{\partial \theta^A} = \frac{\partial J(\boldsymbol{x}_0, t)}{\partial \theta^A} \boldsymbol{a}_i(\boldsymbol{x}_0)$. Accordingly, the gradient of the Jacobian Tensor is the same as shown above for the gradient of the fibre-motion coupling.

Three gradient factors have thus appeared when computing the gradient with respect to motion for the log-probability function: $\frac{\partial \chi(\boldsymbol{x}, t_a, t_b)}{\partial \theta^A}$, $\frac{\partial J(\boldsymbol{x}_0, t)}{\partial \theta^A}$, and $\frac{\partial \nabla \cdot \boldsymbol{v}(\boldsymbol{x}, t)}{\partial \theta^A}$. To explicitly calculate these gradients, the motion must first be parametrised.

### *Motion parameterisation*

In the method according to embodiments of the present invention, parameterising the motion comprises parametrising a velocity field, and then modelling the motion using the parameterised velocity fields. In embodiments, the motion $\chi$ is parameterised by a continuous time-varying (Eulerian) velocity field $\boldsymbol{v}(\boldsymbol{x}, t)$, which ensures that there is diffeomorphic motion when integrated:

$$\chi(\boldsymbol{x}_0, t_a, t_b) = \boldsymbol{x}_a + \int_{t_a}^{t_b} \boldsymbol{v}(\chi(\boldsymbol{x}_a, t_a, t'), t') dt'$$

**Equation 38**

Diffeomorphic motion means that the motion is bijective, differentiable, and has a differentiable inverse, which is a desirable property for most deformable image registration problems, especially for motion-tracking of deformable tissues, since it enforces tissue continuity along time, avoiding degenerate superposition of different regions on the same position.

Turning to describing the velocity field, in some embodiments the velocity field may be described using a dense representation, however this requires regularisation, which is typically in the form of Gaussian smoothing. An alternative representation is to describe the velocity field using a spatio-temporal B-spline diffeomorphic framework. The spatio-temporal B-spline framework parameterises the velocity field using spatio-temporal (4D) B-splines:

$$v^i(\boldsymbol{x}, t) = \rho^{Ii} B_I(\boldsymbol{x}, t) = \rho^{I_1 I_2 I_3 I_4 i} B_{I_1}(x^1) B_{I_2}(x^2) B_{I_3}(x^3) B_{I_4}(t)$$

### Equation 39

where the multi-index notation $I = (I_1, I_2, I_3, I_4)$ is used to allow a compact notation (each index locates a control point, in four dimensions). Each index $I_a$ runs through each of the B-spline control points in the corresponding dimension. The motion is then parameterised by the set of coefficients $\rho^{Ii}$, where the number of coefficients is dependent on the B-spline resolution, that is, the number of B-spline control points. The resultant B-spline parameters $\rho^{Ii}$ can thus be used as motion parameters $\theta^A$.

The B-spline description allows the continuity and smoothness to be controlled both spatially and temporally. In addition, the strain rate tensor (describing the rate of change of deformation), relating to the gradient of the velocity field, can be computed analytically and expressed linearly in the B-spline parameters $\rho^{li}$:

$$\nabla_j v^i(\boldsymbol{x}, t) = \rho^{Ii} \nabla_j B_I(\boldsymbol{x}, t)$$

### Equation 40

The partial derivative of the cubic B-splines is separable and quadratic in the involved coordinate $x^i$. The gradient of $B_I(\boldsymbol{x}, t)$ is therefore:

$$B_I(\boldsymbol{x}, t) = \left(\prod_{i=1}^{3} B_{I_i}(x^i)\right) B_{I_4}(t) \;\Rightarrow\; \nabla_j B_I(\boldsymbol{x}, t) = B'_{I_j}(x^j) \left(\prod_{i=1, i \neq j}^{3} B_{I_i}(x^i)\right) B_{I_4}(t)$$

### Equation 41

These derivatives are required for the velocity divergence, $\nabla_i v^i(\boldsymbol{x}, t)$, appearing in the negative log-probability function (Equation 26).

Returning to Equation 38 and calculating the motion from the velocity field, the integral defining the motion from the velocity field (Equation 38) is numerically integrated. This may be carried out using any of the Runge-Kutta methods of different orders. In the present invention, a first order Euler method is used, as outlined below, however other implementations are possible. Following the Euler method:

$$\chi(\boldsymbol{x}_a, t_a, t_b) \cong \boldsymbol{x}_a + \sum_{\alpha=0}^{m-1} \Delta t \, v(\chi(\boldsymbol{x}_a, t_a, t_a + \alpha\Delta t), t_a + \alpha\Delta t)$$

### Equation 42

The time interval $\Delta t$ is split into m intervals, so that $\Delta t = \frac{t_b - t_a}{m}$ . This can be formulated as the recursion:

$$\chi(\boldsymbol{x}_a, t_a, t' + \Delta t) \cong \chi(\boldsymbol{x}_a, t_a, t') + \Delta t \, v(\chi(\boldsymbol{x}_a, t_a, t'), t')$$

### Equation 43

where $t' = t_a + \alpha\Delta t$ for any $\alpha = 0, \ldots m$ - 1. It should be noted that the recursion is equally valid for integrals forward in time ($\Delta t >$ 0) or backwards in time ($\Delta t < 0$).

Since time resolution of the cine-MRI image is sufficiently high, one interval per image can be used, so that $m = a - b$ (it should be appreciated, however, that more refined integrations may also be used). Thus Equation 43 is simplified, and the iterative formula for describing motion is:

$$\chi(\boldsymbol{x}_a, t_a, t_{b+1}) \cong \chi(\boldsymbol{x}_a, t_a, t_b)) + \Delta t_b v(\chi(\boldsymbol{x}_a, t_a, t_b), t_b)$$

### Equation 44

$\Delta t_b = t_{b+1} - t_b$, which for an equally spaced time sequence (which is typical for cine-MRI images) is $\Delta t_b = T/N$. It should be appreciated that for backwards motion, the recursion is changed so that $\Delta t_b = t_{b-1} - t_b = -T/N$.

### *Gradients with respect to motion - Explicit Calculation*

As shown above, when first deriving the gradients with respect to the motion parameters, the three gradient factors were defined with respect to a set of motion parameters $\theta = \{\theta^A\}$. Once the motion and velocity divergence are represented using the spatio-temporal B-spline diffeomorphic framework (Equations 38 and 39 respectively), the gradient expressions for the motion features are computed explicitly in terms of the B-spline motion parameters, $\rho^{I,j}$. This includes the gradient of the motion function, $\dfrac{\partial \chi(x, t_a, t_b)}{\partial \rho^{I,j}}$, the gradient of the Jacobian tensor, $\dfrac{\partial J(x_0, t)}{\partial \rho^{I,j}}$, and the gradient of the divergence of the velocity field, $\dfrac{\partial \nabla \cdot v(x,t)}{\partial \rho^{I,j}}$.

Firstly, considering the gradient of the motion function, from the iterative formula above (Equation 44), a recursive formula for the gradient can be obtained

$$\frac{\partial \chi^i(x_a, t_a, t_{b+1})}{\partial \rho^{I,j}}$$

$$\cong \frac{\partial \chi^i(x_a, t_a, t_b)}{\partial \rho^{I,j}} + \Delta t_b \delta_j^i B_I(\chi(x_a, t_a, t_b), t_b)$$

$$+ \Delta t_b \nabla_k v^i(\chi(x_a, t_a, t_b), t_b) \frac{\partial \chi^k(x_a, t_a, t_b)}{\partial \rho^{I,j}}$$

$$\cong \left( \delta_k^i + \Delta t_b \nabla_k v^i(\chi(x_a, t_a, t_b), t_b) \right) \frac{\partial \chi^k(x_a, t_a, t_b)}{\partial \rho^{I,j}} + \Delta t_b \delta_j^i B_I(\chi(x_a, t_a, t_b), t_b)$$

**Equation 45**

with the initial conditions $\chi(x_a, t_a, t_a) = x_a$, $\dfrac{\partial \chi(x_a, t_a, t_a)}{\partial \rho^{I,j}} = 0$, and the spatial gradient of the velocity given by Equation 40.

Turning to the gradient of the Jacobian tensor, in order to calculate the gradient, the Jacobian Tensor $J^i_j(x_0, t)$ must first be calculated, where the Jacobian Tensor is given by:

$$J^i_{\ j}(x_0, t) = \frac{\partial \chi^i(x_0, 0, t)}{\partial x_0^j}$$

**Equation 46**

Multiple methods may be used to calculate the Jacobian Tensor, for example numerically, by finite differences, by an appropriate convolutional filter, or using recursion. An example of calculating the Jacobian Tensor and it's gradient using recursion is described below.

Using the equation of motion defined in Equation 38 the following integral formula for the Jacobian can be derived:

$$J^i_{\ j}(x_0, t) = \delta_j^i + \int_0^t \nabla_k v^i(\chi(x_0, t, t'), t') J^k_{\ j}(x_0, t') dt'$$

**Equation 47**

Equation 47 can be approximated with a recursion, simultaneously or after computing motion:

$$J^i_{\ j}(x_0, t_{b+1}) \cong J^i_{\ j}(x_0, t_b) + \Delta t_b \nabla_k v^i(\chi(x_0, 0, t_b), t_b) J^k_{\ j}(x_0, t_b)$$

**Equation 48**

Accordingly, the gradient of the Jacobian with respect to the motion parameters is then also obtained recursively:

$$\frac{\partial J^i{}_j(\boldsymbol{x}_0, t_{b+1})}{\partial \rho^{I,l}} \cong \frac{\partial J^i{}_j(\boldsymbol{x}_0, t_b)}{\partial \rho^{I,l}} + \Delta t_b \delta_l^i \nabla_k B_I(\boldsymbol{\chi}(\boldsymbol{x}_0, 0, t_b), t_b) J^k{}_j(\boldsymbol{x}_0, t_b)$$

$$+ \Delta t_b \nabla_m \nabla_k v^i(\boldsymbol{\chi}(\boldsymbol{x}_0, 0, t_b), t_b) \frac{\partial \boldsymbol{\chi}^m(\boldsymbol{x}_0, t_b)}{\partial \rho^{I,l}} J^k{}_j(\boldsymbol{x}_0, t_b)$$

$$+ \Delta t_b \nabla_k v^i(\boldsymbol{\chi}(\boldsymbol{x}_0, 0, t_b), t_b) \frac{\partial J^k{}_j(\boldsymbol{x}_0, t_b)}{\partial \rho^{I,l}}$$

**Equation 49**

The above recursions, simultaneously with the iterative formula describing the motion (Equation 44) specialised for a = 0, can be performed independently per point $x_0$, without needing to store the full field and allowing its trivial parallelisation.

Finally, considering the gradient of the velocity divergence with respect to the motion parameters $\rho^{I,i} \left( \frac{\partial \nabla \cdot \boldsymbol{v}(\boldsymbol{x},t)}{\partial \rho^{I,i}} \right)$, the divergence of the Eulerian velocity field is first obtained analytically using the spatial gradient of the velocity (Equation 40):

$$\nabla \cdot \boldsymbol{v}(\boldsymbol{x}, t) = \rho^{Ii} \nabla_i B_I(\boldsymbol{x}, t).$$

**Equation 50**

This is linear in motion parameters, and so the gradient is simply

$$\frac{\partial \nabla \cdot \boldsymbol{v}(\boldsymbol{x}, t)}{\partial \rho^{I,i}} = \nabla_i B_I(\boldsymbol{x}, t).$$

**Equation 51**

In this way, the three required gradients with respect to the motion parameters $\rho^{I,i}$ have been computed (Equations 45, 49, and 51).

***Parameterising the fibre architecture***

Returning to Figure 6, once the gradients have been computed with respect to the motion parameters $\rho^{I,l}$, the fibre architecture is parameterised. While parameterising the fibre architecture is shown as occurring after computing the gradients with respect to motion, in some embodiments parameterising the fibre architecture may occur before or in parallel with computing motion gradients.

As discussed previously, at each point $\boldsymbol{x}_0$ of the undeformed configuration of fibres, the local fibre orientation is described by an orthonormal triad, also known as a fibre frame $\{\boldsymbol{a}_i\}$. A fibre frame can be represented and parametrised in different ways, for instance by angle and axis of rotation, by quaternions, or by Euler angles. Any of these representations may be used for the present invention.

In an example of the present invention, Euler angles are used to represent fibre frames, since this is analogous to the parametrization used in RBMs, where the fibre frame is given by the three angles, helical ($\alpha_{he}$), transverse ($\alpha_{tr}$), and sheet ($\beta$) angles, representing the sequence of rotations $\boldsymbol{R}$ aligned with and applied to the heart-adapted fixed reference frame, $\{\boldsymbol{e}_c, \boldsymbol{e}_l, \boldsymbol{e}_t\}$, denoting the circular, longitudinal, and transmural directions following the convention described in Bayer et al 2012 (paper cited previously).

$$\boldsymbol{R}(\alpha_{he}, \alpha_{tr}, \beta) = \boldsymbol{R}_{\boldsymbol{e}_t}(\alpha_{he}) \boldsymbol{R}_{\boldsymbol{e}_l}(-\alpha_{tr}) \boldsymbol{R}_{\boldsymbol{e}_c}(-\beta)$$

**Equation 52**

$$\boldsymbol{a}_1 = \boldsymbol{R}(\alpha_{\mathrm{he}}, \alpha_{\mathrm{tr}}, \beta)\boldsymbol{e}_c,$$

**Equation 53**

$$\boldsymbol{a}_2 = \boldsymbol{R}(\alpha_{\mathrm{he}}, \alpha_{\mathrm{tr}}, \beta)\boldsymbol{e}_l,$$

**Equation 54**

$$\boldsymbol{a}_3 = \boldsymbol{R}(\alpha_{\mathrm{he}}, \alpha_{\mathrm{tr}}, \beta)\boldsymbol{e}_t$$

**Equation 55**

Using Euler angles to parameterise fibre frames allows an intuitive interpretation of the resulting parameters and to reuse part of the method in RBMs.

Expanding the rotations $\boldsymbol{a}_1$, $\boldsymbol{a}_2$, $\boldsymbol{a}_3$ into components, the resulting fibre frame is then written in terms of the myocardium adapted reference frames $\{\boldsymbol{e}_c, \boldsymbol{e}_l, \boldsymbol{e}_t\}$ as:

$$\boldsymbol{a}_1 = \cos\alpha_{\mathrm{he}}\cos\alpha_{\mathrm{tr}}\,\boldsymbol{e}_c + \sin\alpha_{\mathrm{he}}\cos\alpha_{\mathrm{tr}}\,\boldsymbol{e}_l + \sin\alpha_{\mathrm{tr}}\,\boldsymbol{e}_t$$

**Equation 56**

$$\alpha_2 = (\cos\alpha_{2\mathrm{he}}\sin\alpha_{\mathrm{tr}}\sin\beta - \sin\alpha_{\mathrm{he}}\cos\beta)\,\mathbf{e}_c + (\sin\alpha_{\mathrm{he}}\sin\alpha_{\mathrm{tr}}\sin\beta + \cos\alpha_{\mathrm{he}}\cos\beta)\,\mathbf{e}_l - \cos\alpha_{\mathrm{tr}}\sin\beta\,\mathbf{e}_t \qquad \text{Equation 57}$$

$$\alpha_3 = (\cos\alpha_{\mathrm{he}}\sin\alpha_{\mathrm{tr}}\cos\beta + \sin\alpha_{\mathrm{he}}\sin\beta)\,\mathbf{e}_c + (-\sin\alpha_{\mathrm{he}}\sin\alpha_{\mathrm{tr}}\cos\beta + \cos\alpha_{\mathrm{he}}\sin\beta)\,\mathbf{e}_l - \cos\alpha_{\mathrm{tr}}\cos\beta\,\mathbf{e}_t \qquad \text{Equation 58}$$

which provides the fibre frames at each point, $\{\boldsymbol{a}_i(\boldsymbol{x}_0)\}$, from the angles at this same point, $\alpha_{\mathrm{he}}(\boldsymbol{x}_0), \alpha_{\mathrm{tr}}(\boldsymbol{x}_0), \beta(\boldsymbol{x}_0)$.

The spatial variation of the field of frames can also be parametrised in different ways: as a dense field with some regularisation (smoothing) or using a lower dimensional set of parameters based, for instance, on splines. In an embodiment of the present invention, B-splines on the adapted coordinates of the myocardium (circular, longitudinal, transmural) are used to parameterise the spatial variation in the field of frames (consistent with the examples provided above for calculating a PDF for population-based fibre information).

Each control point in the B-spline representation corresponds to a finite-support, base function, $C_L(\boldsymbol{x}_0)$, defined in any point $\boldsymbol{x}_0$ of the myocardium. Analogous to the B-splines parametrising the motion velocity field, the number of control points in each of the adapted coordinates (circular, longitudinal, transmural) controls the corresponding degree of variability of the angles along that coordinate. Thus, the set of parameters for the fibre frame (denoted above generically as $\{\omega^A\}$) will be the set of B-spline control points, $\{\eta^{L,m}\}$, for each of the three Euler angles ($\alpha_{\mathrm{he}}$, $\alpha_{\mathrm{tr}}$, and $\beta$), where L is an index that runs through all control points, and m is an index that runs through each of the angles:

$$\alpha_{\mathrm{he}}(\boldsymbol{x}_0) = \eta^{L,1}C_L(\boldsymbol{x}_0),$$

**Equation 59**

$$\alpha_{\mathrm{tr}}(\boldsymbol{x}_0) = \eta^{L,2}C_L(\boldsymbol{x}_0),$$

**Equation 60**

$$\beta(\boldsymbol{x}_0) = \eta^{L,3}C_L(\boldsymbol{x}_0)$$

**Equation 61**

To compute the gradient of the fibre frame (which describes the fibre orientation at each point) with respect to the fibre orientation parameters the derivative chain rule is applied:

$$\frac{\partial \boldsymbol{a}_i(\boldsymbol{x}_0)}{\partial \eta^{L,1}} = \frac{\partial \boldsymbol{a}_i(\boldsymbol{x}_0)}{\partial \alpha_{\mathrm{he}}(\boldsymbol{x}_0)} C_L(\boldsymbol{x}_0),$$

**Equation 62**

$$\frac{\partial \boldsymbol{a}_i(\boldsymbol{x}_0)}{\partial \eta^{L,2}} = \frac{\partial \boldsymbol{a}_i(\boldsymbol{x}_0)}{\partial \alpha_{\mathrm{tr}}(\boldsymbol{x}_0)} C_L(\boldsymbol{x}_0),$$

**Equation 63**

$$\frac{\partial \boldsymbol{a}_i(\boldsymbol{x}_0)}{\partial \eta^{L,3}} = \frac{\partial \boldsymbol{a}_i(\boldsymbol{x}_0)}{\partial \beta(\boldsymbol{x}_0)} C_L(\boldsymbol{x}_0)$$

**Equation 64**

To obtain the expressions of the appearing derivatives with respect to each of the three Euler angles is straight-forward from Equations 56, 57, and 58.

***Gradients with respect to fibre orientation***

Once the fibre-orientation is parameterised, the gradients with respect to the fibre orientation for the log-probability function can be calculated. The dependence on the fibre orientation parameters appears in three terms in log-probability function: log $\boldsymbol{p}$ (DTI | Fibres, Motion), log $\boldsymbol{p}$(Motion | Fibres), and log $\boldsymbol{p}$ (Fibres | Source$_\mathrm{F}$) (corresponding to subject-specific fibre information, the expression for physical constraints 36, and the population-based fibre information respectively 38).

The first of these terms relates to the subject-specific fibre information, provided, for example, by DTI. The gradient of the DTI term (described in Equation 26) with respect to the fibre orientation parameters $\eta^{L,m}$ is:

$$-\frac{\partial \log p(\mathrm{DTI} \mid \mathrm{Fibres}, \mathrm{Motion})}{\partial \eta^{L,m}} = \frac{2}{\sigma_{\mathrm{DTI}}^2} \sum_t \sum_x \sum_i \lambda_i(\boldsymbol{x}_t, t)\, a_i{}^j(\boldsymbol{x}_t, t) D_{jk}(\boldsymbol{x}_t, t) \frac{\partial a_i{}^k(\boldsymbol{x}_t, t)}{\partial \eta^{L,m}}$$

**Equation 65**

As discussed above, this term only appears if subject-specific DTIs are used as an input. However, in contrast to when computing the gradient with respect to motion, the gradient with respect to fibre orientation is included even if DTI is available for only a single cardiac phase.

Equation 65 includes a gradient with respect to the fibre parameters, $\frac{\partial \boldsymbol{a}_i(\boldsymbol{x}_t,t)}{\partial \eta^{L,m}}$. Computing this gradient shares the same structure as for the equivalent term with respect to the motion parameters ( $\frac{\partial a_i{}^k(\boldsymbol{x}_t,t)}{\partial \theta^A}$ as shown in Equations 32, 33, and 34 above), and uses the angular velocity tensor $\omega_i$:

$$\frac{\partial \boldsymbol{a}_1}{\partial \eta^{L,m}} = -\omega_3 \boldsymbol{a}_2 + \omega_2 \boldsymbol{a}_3,$$

**Equation 66**

$$\frac{\partial \boldsymbol{a}_2}{\partial \eta^{L,m}} = \omega_3 \boldsymbol{a}_1 - \omega_1 \boldsymbol{a}_3,$$

**Equation 67**

$$\frac{\partial \boldsymbol{a}_3}{\partial \eta^{L,m}} = -\omega_2 \boldsymbol{a}_1 + \omega_1 \boldsymbol{a}_2$$

**Equation 68**

again, using

$$\omega_1 = \frac{1}{\|\breve{\boldsymbol{a}}_2\|}\left((\boldsymbol{a}_1 \cdot \tilde{\boldsymbol{a}}_2)\omega_2 - \boldsymbol{a}_3 \cdot \frac{\partial \tilde{\boldsymbol{a}}_2}{\partial \eta^{L,m}}\right), \qquad \omega_2 = \frac{1}{\|\tilde{\boldsymbol{a}}_1\|}\left(\boldsymbol{a}_3 \cdot \frac{\partial \tilde{\boldsymbol{a}}_1}{\partial \eta^{L,m}}\right),$$

$$\omega_3 = -\frac{1}{\|\tilde{\boldsymbol{a}}_1\|}\left(\boldsymbol{a}_2 \cdot \frac{\partial \tilde{\boldsymbol{a}}_1}{\partial \eta^{L,m}}\right)$$

**Equation 69**        **Equation 70**        **Equation 71**

where from Equation 9 $\frac{\partial \tilde{\boldsymbol{a}}_i(\boldsymbol{x}_t,t)}{\partial \eta^{L,m}} = \boldsymbol{J}(\boldsymbol{x}_0,t)\frac{\partial \boldsymbol{a}_i(\boldsymbol{x}_0)}{\partial \eta^{L,m}}$ . Since $\frac{\partial \boldsymbol{a}_i(\boldsymbol{x}_0)}{\partial \eta^{L,m}}$ has been determined above (Equations 62, 63, and 64), the gradient of the DTI with respect to the fibre orientation parameters is obtained.

The second term for which a gradient with respect to fibre orientation parameters is calculated is the fibre-motion coupling term (-log $\boldsymbol{p}$(Motion | Fibres) shown in Equation 26. Calculating the gradient of the fibre-motion coupling term with respect to the fibre orientation parameters gives:

$$-\frac{\partial \log p(\text{Motion} \mid \text{Fibres})}{\partial \eta^{L,m}}$$

$$= \frac{2}{n\sigma_{co}^2}\sum_t \sum_{\boldsymbol{x}_0} \left(\big(\boldsymbol{J}(\boldsymbol{x}_0,t)\boldsymbol{a}_1(\boldsymbol{x}_0)\big)\cdot\big(\boldsymbol{J}(\boldsymbol{x}_0,t)\boldsymbol{a}_2(\boldsymbol{x}_0)\big)\right)\left(\boldsymbol{J}(\boldsymbol{x}_0,t)\boldsymbol{a}_{(1}(\boldsymbol{x}_0)\right)$$

$$\cdot\left(\boldsymbol{J}(\boldsymbol{x}_0,t)\frac{\partial \boldsymbol{a}_{2)}(\boldsymbol{x}_0)}{\partial \eta^{L,m}}\right)$$

**Equation 72**

Equation 72 is again dependent on $\frac{\partial \boldsymbol{a}_i(\boldsymbol{x}_0)}{\partial \eta^{L,m}}$ , and is computed as shown in Equations 62, 63, and 64.

The third term in the log-probability function for which the gradient with respect to fibre parameters is calculated is the population-based fibre information 38 term (term 4 in Equation 26). The generic fibre parameters $\{\omega^A\}$ are replaced by the particular ones, $\{\eta^{L,m}\}$:

$$-\log p(\text{Fibres} \mid \text{Source}_F) = \frac{1}{2}\left(\eta^{L,m} - \overline{\eta^{L,m}}\right)P_{L,m,P,q}\left(\eta^{P,q} - \overline{\eta^{P,q}}\right)$$

**Equation 73**

where $P_{L,m,p,q}$ denotes the precision matrix as in Equation 8 but expressed in the particular parameters. Calculating the gradient of this term:

$$-\frac{\partial \log p(\text{Fibres} \mid \text{Source}_F)}{\partial \eta^{L,m}} = P_{L,m,P,q}\left(\eta^{P,q} - \overline{\eta^{P,q}}\right)$$

**Equation 74**

**Gradient Descent Algorithm**

The gradients outlined above describe the two gradients required for computing the gradient of the log-probability function: the gradient with respect to motion and the gradient with respect to the cardiac fibres:

$$-\frac{\partial \log p(\text{Fibres}, \text{Motion} \mid \text{I}, \text{DTI}, \text{Source}_{\text{F}})}{\partial \rho^{I,i}}$$

**Equation 75**

and

$$-\frac{\partial \log p(\text{Fibres}, \text{Motion} \mid \text{I}, \text{DTI}, \text{Source}_{\text{F}})}{\partial \eta^{L,m}} \; .$$

**Equation 76**

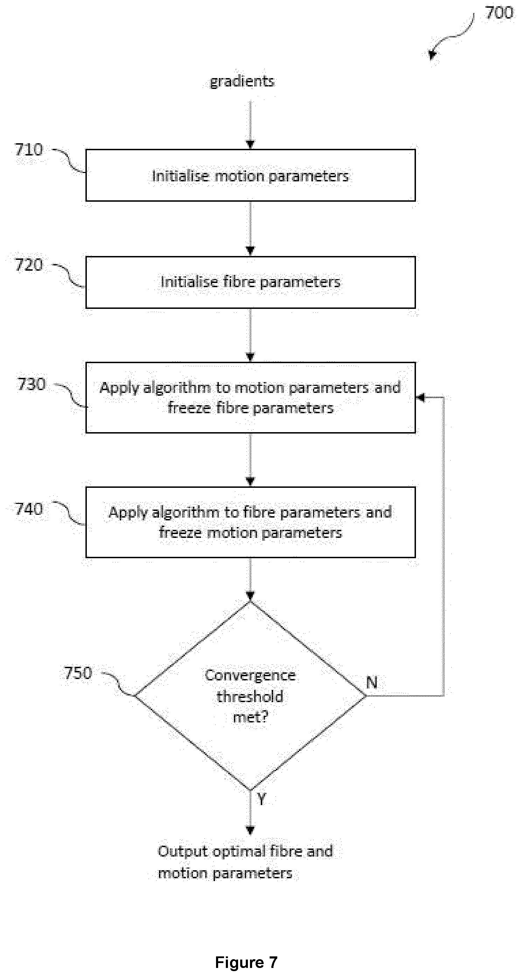

Using these two gradients, any gradient-based optimisation algorithm can be used to optimise the function. Examples of gradient-based optimisation algorithms include gradient descent with constant learning rate, conjugate gradient descent, AdaGrad, stochastic gradient descent, or any of the quasi-Newton methods such as the Broyden-Fletcher-Goldfarb-Shanno (BFGS) algorithm. A flowchart illustrating an example optimisation algorithm 700 is shown in Figure 7.

Firstly, the motion parameters are initialised at Step 710, using a null value $\rho^{li} = 0$, representing a trivial motion, $\chi$ $(x, t_a, t_b) = x.$ It should be appreciated that other initialisation values may be considered if relevant information is available that allows a better initial guess. For instance, if values obtained from a statistical atlas of heart motions are available. The fibre orientation parameters $\eta^{L,m}$ are then also initialised, at Step 720, with a best estimate which maximises $p$(Fibres | Source$_{\text{F}}$). In an embodiment where a Gaussian prior fibre distribution for a population is used as the input, the best prior estimate is the mean of the distribution, $\overline{\eta^{P,q}}$.

The optimisation algorithm 40 is then applied, alternating for the optimisation of the motion and the fibre parameters. The fibre parameters are frozen in their current value, and the gradient descent is applied to optimise the motion parameters at Step 730. Then, the motion parameters are frozen at their current value, and the gradient descent is applied to optimise the fibre parameters at Step 740. The algorithm 40 determines, at Step 750, if a convergence threshold is met, and if so, the algorithm 40 outputs the optimal parameters for the fibre architecture. If the convergence threshold is not met, Steps 730 and 740 are repeated, alternately applying the algorithm 40 to the motion and fibre parameters until the criterion of convergence is satisfied. An alternative approach to achieving convergence could be developed involving joint optimisation of the motion and the fibre parameters.

In some embodiments, a gradient descent with golden section line search may be used as the optimisation algorithm 40. The line search aims at optimising the learning rate for each step, and is a very efficient and thus commonly used line search algorithm.

Once the gradient-descent algorithm 40 is deemed to have minimised the log-probability function (which may be determined, for example, by a pre-determined threshold), the resultant fibre and motion parameters are used to generate a model of the cardiac fibre architecture. The resultant parameters are the optimised parameters, that is, the parameters that best describe the motion and the fibre architecture, satisfying their coupling. The optimised parameters are compatible with the motion observed with the input cine-MRI images and, if available, the subject-specific DTI. The parameterised motion and fibre architecture can then be used to provide a visualisation of cardiac motion, including fibre and sheetlet reorientation with motion.

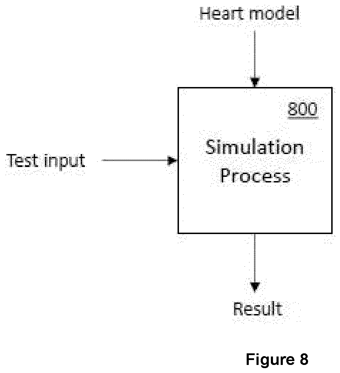

The resultant model may be used for many applications in the field of research and development. For example, the generated heart model may be used to determine the influence of implanted devices on the myocardial fibres, or for testing the effects of drugs on the heart. A specific example of an application for the heart model generated using the method according to the present invention is illustrated in **Figure 8.** As shown, the heart model is input to a simulation process 80, which also receives a test input. An example of a simulation process 80 may be, for example, a simulation for examining impacts and likelihood of success of proposed reconstructive procedures in a diseased heart. The corresponding test inputs may be proposed reconstructive procedures for the heart. In response to receiving a heart model for a patient and a test input, the simulation outputs a result, which, in the example described, would relate to whether the proposed reconstructive procedure is likely to be successful. Using a more realistic heart model for a patient provides more accurate results and allows clinicians to make better choices when determining the reconstructive strategies to use for a patent, thus reducing the risk to patients. For instance, a more accurate motion and fibre estimate allows obtaining better estimates of myocardial local strains, which can be of clinical significance, in particular when pathological or anomalous local contractions are present, such in the case of infarcted tissue. In a related application, the generated optimum parameters for fibre architecture may be used to monitor remodelling of myocardial fibres in a subject after, for example, a myocardial infarction. As more heart models are generated in this manner a heart model that reflects a subset of the population (for example, a population over a certain age or with a certain disease) rather than an individual can be developed using a statistical model of the population. The heart model representing the subset of the population can again be used in research to more accurately test the effect of implantation devices or drugs on the particular subset of the population.

Another potential application for the heart model generated using the method according to the present invention is to combine the heart model with methods that model cardiac muscle function. For example, the heart model may be used for method described in WO2022/106580, which describes a heart simulation method that incorporates muscular electrophysiology and electromechanical properties of the heart. Using the heart model of the present invention, which includes information on cardiac motion and fibre structure, alongside the method described in WO2022/106580, results in a better, more realistic simulation on which to test the effect of, for example, drugs.

This method may also be applied to muscular structures, in particular bodily organs which experience motion that cannot be consciously controlled by a subject. All muscles include myocytes that are oriented according to some fibre architecture, and this fibre architecture will substantially influence their motion. A particularly relevant bodily organ is the uterus - an area of particular interest is contraction of the uterus during birth or during a menstrual period. The same approach of capturing a plurality of images during a muscular motion cycle can be used, and initial models of fibre architecture and of mechanical coupling optimised according to the method to determine a model of fibre architecture consistent with the motion indicated in the plurality of images. This can be used, for example, to study the relationship between uterus motion and inflammation or pain (dysmenorrhea).

While the primary use of this method described above is in providing a better model of the fibre architecture, a further use is to improve imaging - in the case of DTI image capture, DTI imaging. One exemplary case of this in the context of DTI cardiac imaging is described below.

In the conventional case, the presence of multiple cardiac phases can be problematic for establishing high resolution fibre images, as there is not an effective way to combine information from images obtained in different cardiac phases. In order to obtain higher resolution, it would be normal to devise an acquisition sequence in which all image slices were obtained in the same cardiac phase. That would necessarily lead to an extended period of time being required for DTI image capture. Moreover, in addition to making image capture more time consuming, such an approach may introduce additional complexity if there are progressive changes in cardiac behaviour over multiple cardiac cycles. However, using a method as described here, cardiac motion can be described from images obtained from multiple cardiac phases, and this information can be used to enhance their resolution even though the images are from different cardiac phases. As a model of cardiac motion is available, by applying the inverse motion to all DTI images at different cardiac phases, these images can be explicitly fused into a single 3D DTI image of higher resolution. In effect, the ability to transform the images for the determined motion enable the type of fusion and super resolution strategies customarily applied to scalar images, such as cine-MRI, to tensorial DTI images.

**Claims**

1.  A method of estimating muscle fibre architecture for a muscular structure, the method comprising:

    receiving a plurality of images that represent the motion of the muscular structure during a motion sequence;
    receiving an initial model of muscle fibre architecture;
    receiving a model of mechanical coupling between muscular structure motion and muscle fibres;
    generating a joint function using muscular structure motion as represented in the plurality of images, the initial model of muscle fibre architecture, and the model of mechanical coupling;
    applying an optimisation procedure to optimise the joint function; and
    from the optimisation procedure, determining a model of muscle fibre architecture consistent with the muscular structure motion indicated in the plurality of images.

2.  The method of Claim 1, wherein the optimisation procedure comprises:

    parameterising the muscular structure motion indicated in the plurality of images to determine a set of motion parameters;
    parameterising the fibre architecture indicated in the initial model of muscle fibre architecture to determine a set of fibre architecture parameters; and

optimising the joint function by alternatively optimising the set of motion parameters and the set of fibre architecture parameters until convergence is reached.

3. The method of Claim 2, wherein optimising the set of motion parameters and the set of fibre architecture parameters comprises:

   calculating the gradient of the joint function with respect to the set of motion parameters and the set of fibre architecture parameters; and
   applying a gradient - based optimisation algorithm to the joint function.

4. The method of Claim 2 or 3, wherein determining the model of muscle fibre architecture comprises using the converged set of motion parameters and set of fibre architecture parameters.

5. The method of any of Claims 2 to 4, wherein the muscular structure motion is parameterised using a continuous time-varying velocity field.

6. The method of any of Claims 2 to 5, wherein parametrising the muscle fibre architecture comprises parameterising the local fibre orientation and parameterising the spatial variation in fibre orientation.

7. The method of any of Claims 1 to 6, wherein the function describing motion, the initial model of muscle fibre architecture and the model of mechanical coupling are probabilistic models.

8. The method of any of Claims 1 to 7, wherein the method additionally comprises generating a conditional probability density function to represent muscular structure motion as indicated in the plurality of images, for the initial model of muscle fibre architecture and for the model of mechanical coupling.

9. The method of Claim 8, wherein the joint function is generated using the probability density functions.

10. The method of any of Claims 1 to 9, wherein the plurality of images are received from a cine-MRI.

11. The method of any of Claims 1 to 10, wherein the initial model of muscle fibre architecture is population-based fibre information or fibre information provided from rule-based models.

12. The method of any of Claims 1 to 11, wherein the model of mechanical coupling is derived from strain properties of muscle fibres.

13. The method of any of Claims 1 to 12, wherein the method additionally comprises:

   receiving muscle fibre information for an individual;
   generating a function describing the muscle fibre information for the individual; and
   generating the joint function using muscular structure motion as represented in the plurality of images, the initial model of muscle fibre architecture, the model of mechanical coupling, and the function describing the muscle fibre information for the individual.

14. The method of Claim 13, wherein the muscle fibre information for the individual comprises at least one diffuse tensor image, and the function describing the myocardial fibre information for the individual is a conditional probability density function.

15. The method of any preceding claim, wherein the muscular structure is a heart, the muscle fibre architecture is a myocardial fibre architecture, and the motion sequence is a cardiac cycle.

16. A method of modelling function of a heart, comprising a method of estimating myocardial fibre architecture as claimed in claim 15.

17. The method of any of claims 1 to 14, wherein the muscular structure is a uterus.

18. A method of visualizing muscular motion, the method comprising estimating muscle fibre architecture for a muscular structure according to any of claims 1 to 17 and using the optimised set of motion parameters and the set of fibre architecture parameters to provide the visualization.

19. A method of image enhancement in imaging a muscular structure, comprising:

estimating muscle fibre architecture as claimed in any of claims 1 to 14;

determining the muscular structure motion during the motion sequence;

applying an inverse motion parameter to at least some of the plurality of images to provide images compensated for the muscular structure motion; and

combining the compensated images using image fusion or image super resolution to provide at least one higher resolution image of the muscular structure.

20. A system for estimating muscle fibre architecture using a plurality of images indicating motion of a muscular structure, the system comprising:

an input engine for receiving each of the plurality of images indicating muscular structure motion;

a function creator configured to generate a joint function using muscular structure motion as represented in the plurality of images, an initial model of muscular fibre architecture, and a model of mechanical coupling between muscular structure motion and muscular fibres;

an optimisation engine configured to optimise the joint function; and

a model generator configured to determine, using the optimised joint function, a model of muscular fibre architecture consistent with the muscular structure motion indicated in the plurality of images.

Figure 1

Figure 2

300

310 — Receive image sequence describing motion

320 — *Optional*
Receive subject-specific fibre information (e.g. DTI)

330 — Retrieve prior fibre information and expression for physical constraints

340 — Process inputs

350 — Apply optimisation procedure

360 — Generate model of the cardiac fibre architecture using optimised parameters

**Figure 3**

400

410 — Calculate PDF for each input

420 — Combine PDFs into a posterior probability function

430 — Create log-probability function

**Figure 4**

500

Log-probability function

↓

510 — | Calculate gradient of log-probability function |

↓

520 — | Apply gradient descent algorithm |

↓

Optimal fibre and
motion parameters

**Figure 5**

600

Log-probability function

610 — Compute gradients with respect to motion for the log-probability function

620 — Parameterise motion using B-splines to obtain motion parameters $\rho^{I,j}$

630 — Compute gradients with respect to motion explicitly in terms of B-spline motion parameters, $\rho^{I,j}$.

640 — Compute gradients with respect to fibre orientation for the log-probability function

650 — Parameterise fibre architecture using Euler angles and B-splines to obtain fibre parameters $\eta^{L,m}$

660 — Calculate gradients with respect to fibre orientation explicitly in terms of fibre parameters $\eta^{L,m}$

670 — Apply gradient-descent optimisation algorithm

optimal fibre and motion parameters

**Figure 6**

700

gradients

710 — Initialise motion parameters

720 — Initialise fibre parameters

730 — Apply algorithm to motion parameters and freeze fibre parameters

740 — Apply algorithm to fibre parameters and freeze motion parameters

750 — Convergence threshold met?    N

Y

Output optimal fibre and motion parameters

**Figure 7**

Figure 8

920

940

960

Figure 9a

Figure 9b

**Figure 9c**

| | Europäisches Patentamt<br>European Patent Office<br>Office européen des brevets | **EUROPEAN SEARCH REPORT** | **Application Number**<br>EP 24 38 2251 |
|---|---|---|---|

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SIKDAR SIDDHARTHA ET AL: "Dynamic Ultrasound Imaging Applications to Quantify Musculoskeletal Function", EXERCISE AND SPORT SCIENCES REVIEWS, vol. 42, no. 3, 1 July 2014 (2014-07-01), pages 126-135, XP093191142, US ISSN: 0091-6331, DOI: 10.1249/JES.0000000000000015 * page 15 * * paragraph [0005] * * page 2, paragraphs 1, 3 * * page 6, paragraph 2 * | 1-20 | INV.<br>G16H30/00 |
| A | Radomir Chabiniok: "Personalized Biomechanical Heart Modeling for Clinical Applications", , 24 January 2011 (2011-01-24), XP055415108, Retrieved from the Internet: URL:https://tel.archives-ouvertes.fr/tel-00839929/document [retrieved on 2017-10-12] * the whole document * | 1-20 | |
| Y | LIU HUAFENG ET AL: "Spatiotemporal Strategies for Joint Segmentation and Motion Tracking From Cardiac Image Sequences", IEEE JOURNAL OF TRANSLATIONAL ENGINEERING IN HEALTH AND MEDICINE, vol. 5, 23 February 2017 (2017-02-23), pages 1-19, XP011644750, DOI: 10.1109/JTEHM.2017.2665496 [retrieved on 2017-04-04] * paragraph [0C.1] * | 16 | |

TECHNICAL FIELDS SEARCHED (IPC)

G16H

-/--

The present search report has been drawn up for all claims

| Place of search | Date of completion of the search | Examiner |
|---|---|---|
| Munich | 22 August 2024 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................
& : member of the same patent family, corresponding document

EPO FORM 1503 03.82 (P04C01)

page 1 of 2

| | Europäisches Patentamt European Patent Office Office européen des brevets | **EUROPEAN SEARCH REPORT** | **Application Number** EP 24 38 2251 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANGBIN DING ET AL: "Aligning Multi-Sequence CMR Towards Fully Automated Myocardial Pathology Segmentation", ARXIV.ORG, CORNELL UNIVERSITY LIBRARY, 201 OLIN LIBRARY CORNELL UNIVERSITY ITHACA, NY 14853, 7 February 2023 (2023-02-07), XP091431110, * page 4, column 1, paragraph 4 * ----- | 1-20 | |
| A | Yun Zhu: "LV Segmentation and Motion Analysis from 4D Cardiac Images", , 1 June 2010 (2010-06-01), XP055118701, Retrieved from the Internet: URL:http://media.proquest.com/media/pq/cla ssic/doc/2084419451/fmt/ai/rep/SPDF?hl=&ci t:auth=Zhu, Yun&cit:title=LV segmentation and motion analysis from four-dimensional cardiac images&cit:pub=ProQuest Dissertations and Theses&cit:vol=&cit:iss=&cit:pg=n/a&cit:da te=2010&ic=true&cit:prod=ProQuest Dissertati [retrieved on 2014-05-19] * the whole document * ----- | 1-20 | **TECHNICAL FIELDS SEARCHED    (IPC)** |

The present search report has been drawn up for all claims

| Place of search | Date of completion of the search | Examiner |
|---|---|---|
| Munich | 22 August 2024 | Laub, Christoph |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022106580 A

**Non-patent literature cited in the description**

- **FERREIRA et al.** In vivo cardiovascular magnetic resonance diffuse tensor imaging shows evidence of abnormal myocardial laminar orientations and mobility in hypertrophic cardiomyopathy. *Journal of Cardiovascular Magnetic Resonance*, 2014, vol. 16 (1), 87

- **BAYER, J. D. ; BLAKE, R. C. ; PLANK, G. ; TRAYANOVA, N. A.** A novel rule-based algorithm for assigning myocardial fiber orientation to computational heart models. *Annals of biomedical engineering*, 2012, vol. 40, 2243-2254
- **BAYER, J. ; PRASSL, A. J. ; PASHAEI, A.** Universal ventricular coordinates: A generic framework for describing position within the heart and transferring data. *Medical image analysis*, 2018, vol. 45, 83-93